# EUROPEAN PATENT SPECIFICATION

(11) **EP 1 499 291 B1**
(45) Date of publication and mention of the grant of the patent: **07.01.2009**
(21) Application number: 03728526.9
(22) Date of filing: 25.04.2003
(51) Int. Cl.: A61K 9/00, A61K 9/22, A61P 25/04

(54) **METHODS AND DOSAGE FORMS FOR CONTROLLED DELIVERY OF OXYCODONE**
METHODEN UND DARREICHUNGSFORMEN ZUR KONTROLLIERTEN FREISETZUNG VON OXYKODON
PROCÉDÉS ET FORMES DOSIFIÉES POUR L'ADMINISTRATION CONTRÔLÉE D'OXYCODONE

(30) Priority: 29.04.2002 US 376470 P
(43) Date of publication of application: 26.01.2005
(73) Proprietor: ALZA Corporation, Mountain View, CA 94039-7210 (US)
(72) Inventor: AYER, Atul, Palo Alto, CA 94303 (US); SHIVANAND, Padmaja, Mountain View, CA 94040 (US); MODI, Nishit, B., Sunnyvale, CA 94087 (US); SEROFF, Sonya, San Jose, CA 95130 (US); DESJARDIN, Michael, A., Sunnyvale, CA 94087 (US); FINK, Tracy, A., Campbell, CA 95008 (US); HEARNEY, Linda, M., Saratoga, CA 95070 (US); JOHNSON, Deborah, J., Palo Alto, CA 94036 (US); ALLPHIN, Clark, P., Mountain View, CA 94043 (US)
(74) Representative: Williams, Paul Edwin
(86) International application number: PCT/US2003/012781
(87) International publication number: WO 2003/092648

(56) References cited:
- WO-A-02/087512
- US-A1- 2001 038 856
- HAGEN NEIL A ET AL: "Comparative clinical efficacy and safety of a novel controlled-release oxycodone formulation and controlled-release hydromorphone in the treatment of cancer pain." CANCER, vol. 79, no. 7, 1997, pages 1428-1437, XP002249725 ISSN: 0008-543X
- SANTUS G ET AL: "Osmotic drug delivery: a review of the patent literature" JOURNAL OF CONTROLLED RELEASE, ELSEVIER SCIENCE PUBLISHERS B.V. AMSTERDAM, NL, vol. 35, no. 1, 1 July 1995 (1995-07-01), pages 1-21, XP004037488 ISSN: 0168-3659 cited in the application

## Description

### FIELD OF THE INVENTION

This invention pertains to the use of oxycodone in the manufacture of dosage forms. In particular, the invention is directed to the use of oxycodone in the manufacture of a sustained release oral dosage form for the management of pain.

### BACKGROUND OF THE INVENTION

Oxycodone is an analgesic with its principal therapeutic effect the relief of pain. Oxycodone is indicated for the relief of moderate to severe pain such as pain due to surgery, cancer, trauma, biliary colic, renal colic, myocardial infarction and bums. A pharmaceutically acceptable dosage form for oral administration of oxycodone to provide analgesic therapy beyond its short half-life at a controlled rate over an extended period of time appears to be lacking In the pharmaceutical and medical arts.

The pharmacological and medical properties of analgesic opioids including oxycodone are known in Pharmaceutical Sciences, Remington, 17th Ed., pp. 1099-1044 (1985); and The Pharmacological Basis of Therapeutics, Goodman and Rail, 8th Ed., pp. 485-518 (1990). Generally, the analgesic action of parenterally administered oxycodone is apparent within 15 minutes, while the onset of action of orally administered oxycodone is somewhat slower with analgesia occurring within about 30 minutes. In human plasma the half-life of orally administered immediate release oxycodone is about 3.2 hours. Physicians' Desk Reference, Thompson Healthcare, 56th Ed., pp. 2912-2918 (2002).

Prior to this invention, oxycodone was administered in conventional forms, such as a nonrate-controlling, dose-dumping immediate release tablet, or by a dose-dumplng capsule, and usually at multiple, repetitive dosing intervals throughout the day. Oxycodone is also administered on a twice-a-day basis with a controlled release matrix system, Oxycontin^{®}**.** The Oxycontin^{®} mode of therapy, however, continues to lead to an initial high dose of oxycodone In the blood after administration, followed by a decreased levels of oxycodone in the blood. Moreover, this peak and trough occurs twice during a 24-hour period due to the twice-a-day dosing regimen. The concentration differences in dosing patterns are related to the presence and absence of administered drug, which is a major disadvantage, associated with these prior dosage forms. Conventional dosage forms and their mode of operation, including dose peaks and valleys, are discussed in Pharmaceutical Sciences, Remington, 18th Ed., pp. 1676-1686 (1990), Mack Publishing Co.; The Pharmaceutical and Clinical Pharmacokinetics, 3rd Ed., pp. 1-28 (1984), Lea and Febreger, Philadelphia; and in U.S. Patents Nos. 3,598,122 and 3,598,123, both issued to Zaffaroni.

Purdue Pharma presently markets an extended release oral dosage form of oxycodone, Oxycontin^{®} represented by US Pat. No. 5,672,360. While Oxycontin^{®} is indicated for administration twice a day, the patent discloses a "once-a-day' "oral sustained release dosage form" containing oxycodone described as achieving maximum blood plasma concentration from 2 to 10 hours after administration that Is more than twice the blood plasma concentration 24 hours after administration. However, such a blood plasma concentration profile continues to exhibit nothing more than a delayed first order delivery rate similar to an immediate release dosage form having a single ascent to a single peak concentration followed by a steady decline in concentration from the peak when the release rate of oxycodone from the dosage form diminishes.
Cancer (Hagen et al. 1997, Vol. 79, No. 7, pages 1.428-1437) describes studies performed using Oxycontin^{®} from Purdue Pharma to compare controlled-release oxycodone with controlled-release hydromorphone. The Oxycontin^{®} system is for administration twict-a-day, providing an initial high dose of oxycodone in the blood after administration, followed by decreased levels of oxycodone in the blood. This "peak and trough" pattern occurs twice during a 24 hour period due to the twice-a-day dosing regimen. The concentration differences in dosing patterns are related to the presence and absence of administered drug, which is a major disadvantage with such dosage forms.

The continued drawback of such a plasma concentration profile is that it continues to provide a significant peak and trough of analgesic therapy throughout the day. The peak concentration, as with immediate release dosage forms, is higher than therapeutically necessary and the ensuing trough provides lower than therapeutically beneficial treatment to a patient. Such a profile continues to result in similar side effects to immediate release dosage forms. Tamely, sedation from over medicating at the peak concentration and breakthrough pain as the concentration falls below the efficacious level toward during a 24 hour dosing regimen. Physicians' Desk Reference, Thompson Healthcare, 56th Ed., pp. 2912-2918 (2002).

Other patents relating to Oxycontin^{®} include US Pat. Nos. 4,881,598; 4,970,075; 5,226,331; 5,508,042; 5,649,912; and 5,656,295. These patents disclose similar extended release dosage forms for delivery over 12 hours and do not disclose once-a-day dosing.
The art is further replete with descriptions of dosage forms for the controlled release of pharmaceutical agents.
For example, WO 02/087512 A2, which was published after the priority date of the present application but which itself has an earlier priority date, discloses examples of oxycodone sustained release osmotic tablets, and US 2001/0038856 A1 discloses controlled release dosage forms comprising hydromorphone for the management of pain.
While a variety of sustained release dosage forms for delivering certain drugs exhibiting short half-life may be known, not every drug may be suitably delivered from those dosage forms because of solubility, metabolic processes, absorption and other physical, chemical and physiological parameters that may be unique to the drug and the mode of delivery.
Additionally, side effects associated with oxycodone, such as sedation, tolerance, constipation, appear to be related to high blood plasma concentration levels restricting the ability to administer a single daily immediate release dose.
Devices in which a drug composition is delivered as a slurry, suspension or solution from a small exit orifice by the action of an expandable layer are described in U. S. Patents Nos. 5,633,011; 5,190,765; 5,252,338; 5,620,705; 4,931,285; 5,006,346; 5,024,842; and 5,160,743. Typical devices include an expandable push layer and a drug layer surrounded by a semipermeable membrane. In certain instances, the drug layer is provided with a subcoat to delay release of the drug composition to the environment of use or to form an annealed coating in conjunction with the semipermeable membrane.

Devices in which a drug composition is delivered in a dry state from a large exit orifice by the action of an expandable layer are described in US Patent Nos. 4,892,778, 4,915,949 and 4,940,465. Those references describe a dispenser for delivering a beneficial agent to an environment of use that includes a semipermeable wall containing a layer of expandable material that pushes a dry drug layer out of the compartment formed by the wall. The exit orifice in the device is substantially the same diameter as the inner diameter of the compartment formed by the wall.

While dosage forms delivering the drug composition to the environment of use in the dry state may provide suitable release of drug over a prolonged period of time, the exposure of the drug layer to the environment of use may result in agitation-dependent release of drug that in some circumstances is difficult to control. Accordingly, it may be advantageous to release the drug as a slurry or suspension that may be metered by control of rate of expansion of the push layer and the size of the exit orifice in the dosage form as in accordance with this invention.

There remains a need for effective dosing methods, dosage forms and devices that will permit the controlled release of the aforementioned compound over a prolonged period of time to reduce the amount of the active agent that the patient is exposed to at any particular time and to increase the time between dosing, preferably to obtain a once-a-day dosing regimen.
US 2001 038856 A1 discloses osmotic once-a-day hydromorphone dosage forms.

### Summary of the Invention

According to the present invention there is provided a sustained release oral dosage form for once-a-day controlled delivery of oxycodone comprising:
(a) a core which comprises:
   (i) an osmotic agent; and
   (ii) oxycodone and/or one or more pharmaceutically-acceptable acid addition salts thereof (the compound);
(b) a semipermeable membrane enveloping the core;
(c) an exit orifice through the semipermeable membrane which communicates with the core so as to allow release of the compound to the environment; and
(d) an immediate release coating which comprises 0.5 to 75mg of the compound and 0.5 to 275mg of a pharmaceutically acceptable carrier selected from the group consisting of alkylcellulose, hydroxyalkylcellulose and hydroxypropylalkylcellulose.

Preferable the core of the sustained release oral dosage form comprises a polyalkylene oxide.

Preferably the core comprises a first drug layer which comprises the compound, and a second expandable layer which comprises the osmotic agent and does not comprise the compound.

The prior art did not appreciate that oxycodone can be made into a cont nuous-release dosage form or into a therapeutic composition as claimed herein that provides efficacious analgesic therapy over 24 hours. The prior art did not appreciate a dosage form and a therapeutic composition can be made available comprising an osmogel, such as a polyalkylene oxide, and other ingredients such as an osmagent-that reduce the peak and trough delivery associated with side effects and breakthrough pain.

The prior art does not make obvious oxycodone formulated with a polyalkylene oxide, as the mechanism that controls the release of oxycodone from polyalkylene oxide is complex. For example, the oxycodone could become immobile and trapped in the polyalkylene oxide; also, the polyalkylene oxide could exhibit unacceptable swelling in the presence of aqueous, including biological, fluid and thereby change the rate of release of the oxycodone from the polyalkylene oxide. Further, the osmogel, such as polyalkylene oxide, can possess a glass-transition temperature below human body temperature, which leads away from using oxycodone in such an environment. Additionally, the properties of oxycodone and polyalkylene oxide exemplified by the crystalinity of oxycodone in polyalkylene oxide, the burst or lag effect of oxycodone in polyalkylene oxide, and the oxycodone solubility in a polyalkylene oxide hydrogel, all attest to the nonobviousness of the present invention.

The above presentation dictates the critical need for a dosage form and for a therapeutic composition that overcomes the shortcomings of conventional dosage forms and controlled release matrix forms, including tablets, capsules, elixir and suspensions. These conventional dosage forms and their accompanying peaks and valleys in blood plasma concentration do not provide for optimal dose-regulated drug therapy over an extended period of time. Oxycodone as delivered by the prior art is dosed two or more times a day, which does not lend itself to controlled and sustained therapy. This prior-art pattern of drug administration indicates the need for a dosage form and for a therapeutic composition that can administer oxycodone in a rate-controlled dose over an extended period of time to provide constant therapy, and eliminate the blood plasma concentration peaks, valleys and multiple dosing of the prior art. This application describes an oral, relatively easy to administer mode and manner of oxycodone.

### BRIEF DESCRIPTION OF THE FIGURES

The following figures are not drawn to scala.

Figure 1 illustrates one example of a dosage form described herein, illustrating the dosage form prior to administration to a subject

Figure 2 illustrates the dosage form of Figure 1 in opened section, depicting a dosage form comprising an internally housed, pharmaceutically acceptable therapeutic oxycodone composition.

Figure 3 illustrates an opened view of drawing Figure 1, illustrating a dosage form internally comprising a oxycodone composition and a separate and contacting displacement composition comprising means for pushing the pharmaceutical oxycodone composition from the dosage form.

Figure 4 illustrates a dosage form which further includes an instant-release external overcoat of oxycodone on the dosage form.

Figure 5 models the mean plasma oxycodone concentration profile for a single 20 mg dose over 24 hours with a 3 mg oxycodone overcoat and 17 mg oxycodone core.

Figure 6 models the mean plasma oxycodone concentration profile for a single 20 mg dose over 24 hours at steady state with a 3 mg oxycodone overcoat and 17 mg oxycodone core.

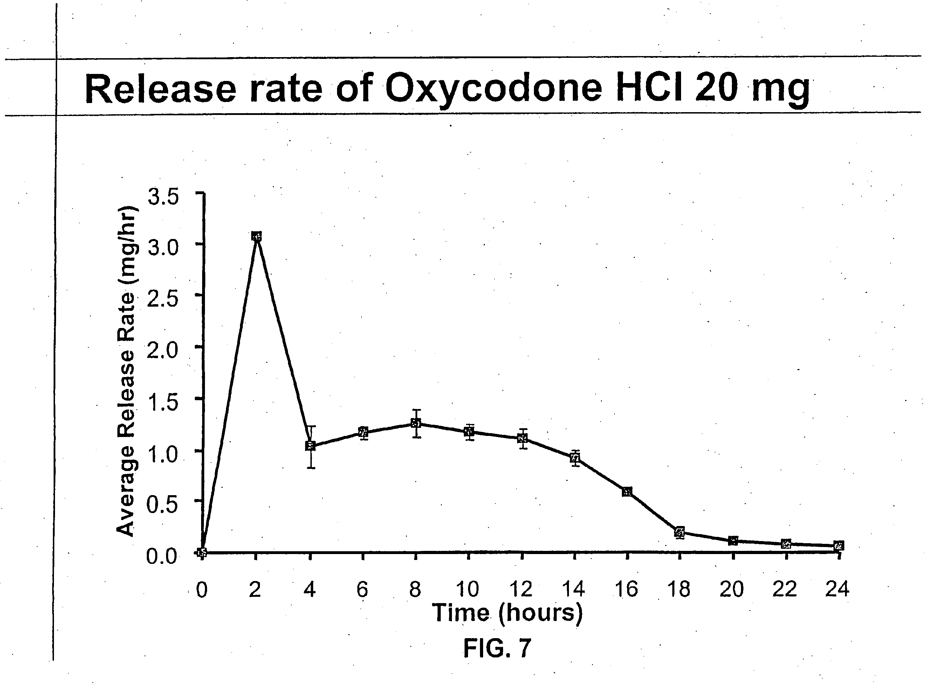

Figure 7 illustrates an average release rate profile (release rate as a function of time) from a 20 mg oxycodone dosage form having the general characteristics illustrated in Figure 4, with a 3 mg oxycodone overcoat and 17 mg oxycodone core;

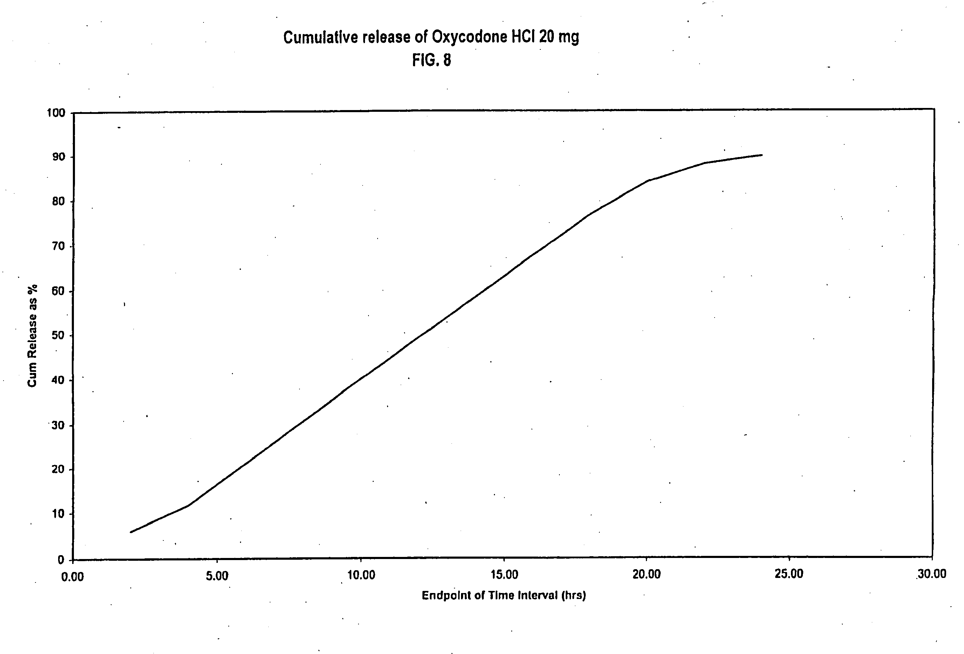

Figure 8 illustrates the cumulative release of oxycodone over time from a representative 20 mg oxycodone dosage form having the general characteristics illustrated in Figure 4 with a 1 mg oxycodone overcoat and 19 mg oxycodone core;

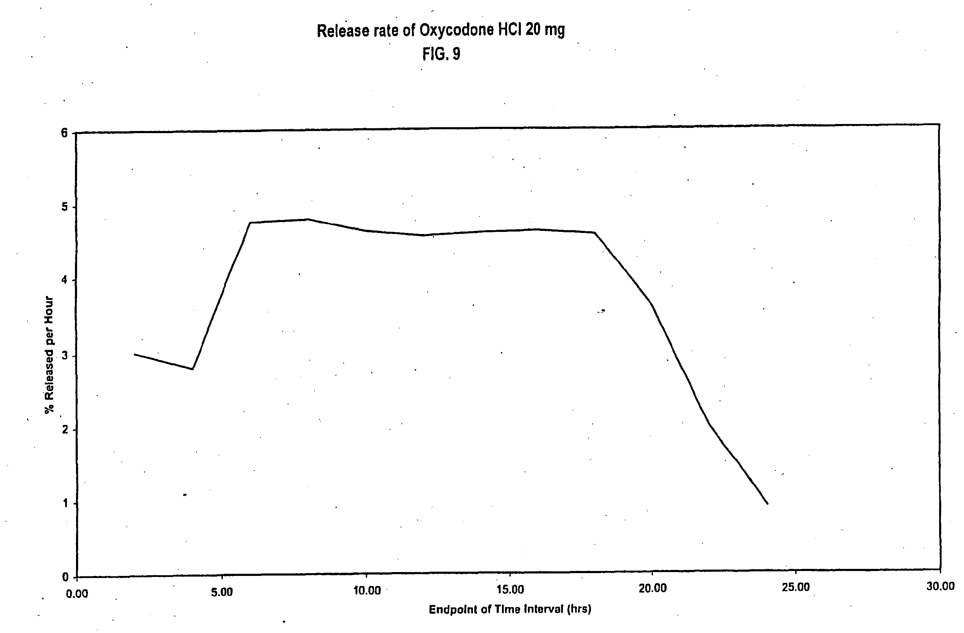

Figure 9 illustrates the percent released per hour release profile (releases rate as a function of time) of oxycodone for 20 mg dosage form having the general characteristics illustrated in Figure 4 with a 1 mg oxycodone overcoat and 19 mg oxycodone core;

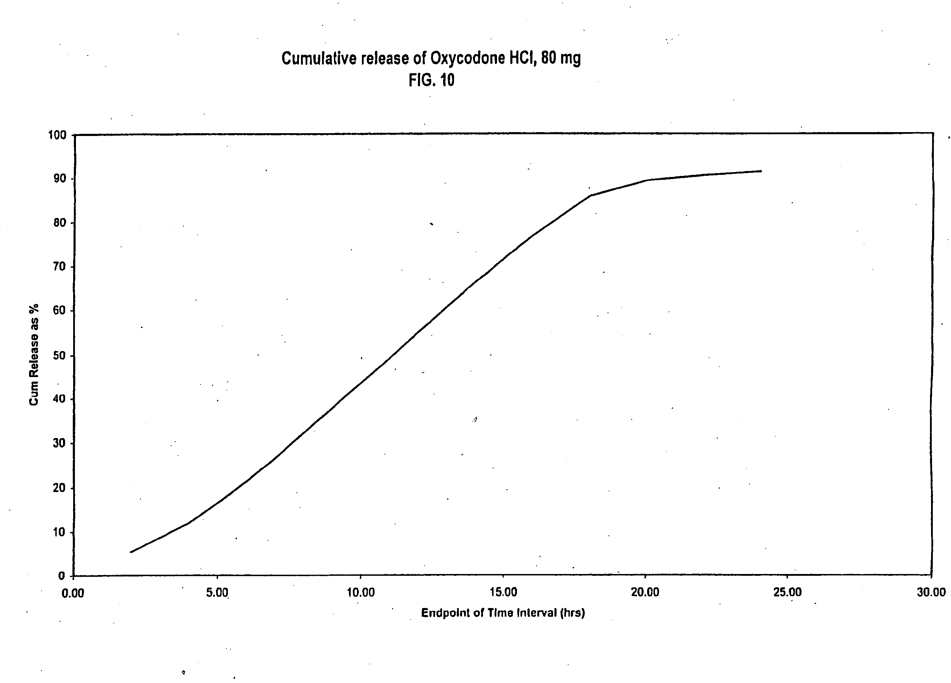

Figure 10 illustrate the cumulative release of oxycodone over time from a representative 80 mg oxycodone dosage form having the general characteristics illustrated in Figure 4 with a 4 mg oxycodone overcoat and 76 mg oxycodone core;

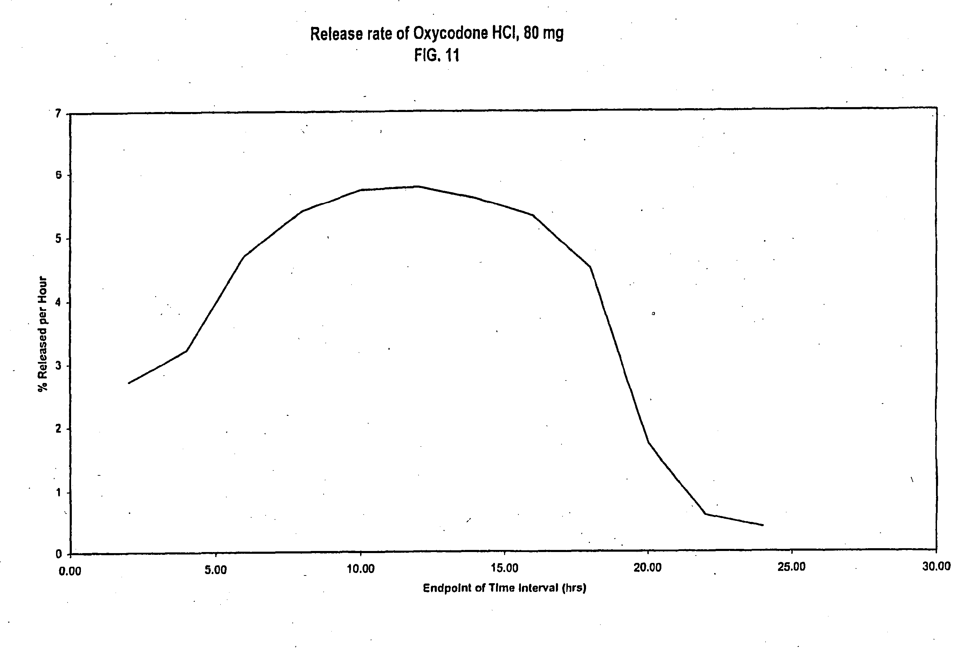

Figure 11 illustrates the percent released per hour release profile (release rate as a function of time) of oxycodone for 80 mg dosage form having the general characteristics illustrated in Figure 4 with a 4 mg oxycodone overcoat and 76 mg oxycodone core;

In the drawing figures and specification, like parts in related figures are identified by like numbers. The terms appearing earlier in the specification and in the description of the drawing figures, as well as examples thereof, are further described elsewhere in the disclosure.

### DETAILED DESCRIPTION OF THE INVENTION

The present invention is best understood by reference to the following definitions, the drawings and exemplary disclosure, provided herein.

### Definitions

By "dosage form" is meant a pharmaceutical composition or device comprising an active pharmaceutical agent, such as oxycodone or a pharmaceutically-acceptable acid addition salt thereof, the composition or device optionally containing inactive ingredients, i.e., pharmaceutically acceptable excipients such as suspending agents, surfactants, disintegrants, binders, diluents, lubricants, stabilizers, antioxidants, osmotic agents, colorants, plasticizers, coatings and the like, that are used to manufacture and deliver active pharmaceutical agents.

By "active agent", "drug", or "compound" is meant an agent, drug, or compound having the characteristics of oxycodone or a pharmaceutically-acceptable acid addition salt thereof.

By "pharmaceutically-acceptable acid addition salt" or "pharmaceutically acceptable salt", which are used interchangeably herein, are meant those salts in which the anion does not contribute significantly to the toxicity or pharmacological activity of the salt, and, as such, they are the pharmacological equivalents of the bases of the oxycodone compound. Examples of pharmaceutically acceptable acids that are useful for the purposes of salt formation include but are not limited to hydrochloric, hydrobromic, hydroiodic, citric, acetic, benzoic, mandelic, phosphoric, nitric, mucic, isethionic, palmitic, and others.

By "sustained release " is meant predetermine continuous release of active agent to an environment over a prolonged period.

The expressions "exit," "exit orifice," "delivery orifice" or "drug delivery orifice," and other similar expressions, as may be used herein include a member selected from the group consisting of a passageway; an aperture; an orifice; and a bore. The expression also includes an orifice that is formed or formable from a substance or polymer that erodes, dissolves or is leached from the outer wall to thereby form an exit orifice.

A drug "release rate" refers to the quantity of drug released from a dosage form per unit time, e.g., milligrams of drug released per hour (mg/hr). Drug release rates for drug dosage forms are typically measured as an *in vitro* rate of dissolution, i.e., a quantity of drug released from the dosage form per unit time measured under appropriate conditions and in a suitable fluid. The dissolution tests utilized in the Examples described herein were performed on dosage forms placed in metal coil sample holders attached to a USP Type VII bath indexer in a constant temperature water bath at 37°C. Aliquots of the release rate solutions were injected into a chromatographic system to quantify the amounts of drug released during the testing intervals.

By "release rate assay" is meant a standardized assay for the determination of the release rate of a compound from the dosage form tested using a USP Type 7 interval release apparatus. It is understood that reagents of equivalent grade may be substituted in the assay in accordance with generally accepted procedures.

For clarity and convenience herein, the convention is utilized of designating the time of drug administration as zero hours (t = 0 hours) and times following administration in appropriate time units, e.g., t = 30 minutes or t = 2 hours, etc.

As used herein, unless otherwise specified, a drug release rate obtained at a specified time "following administration" refers to the *in vitro* drug release rate obtained at the specified time following implementation of an appropriate dissolution test. The time at which a specified percentage of the drug within a dosage form has been released may be referenced as the "Tₓ" value, where "x" is the percent of drug that has been released. For example, a commonly used reference measurement for evaluating drug release from dosage forms is the time at which 70% of drug within the dosage form has been released. This measurement is referred to as the "T₇₀" for the dosage form.

An "immediate-release dosage form" refers to a dosage form that releases drug substantially completely within a short time period following administration, i.e., generally within a few minutes to about 1 hour.

By "sustained release dosage form" is meant a dosage form that releases drug substantially continuously for many hours. Sustained release dosage forms described herein exhibit T₇₀ values of at least about 10 to 20 hours and preferably 15 to 18 hours and more preferably about 17 hours or more. The dosage forms continuously release drug for sustained periods of at least about 10 hours, preferably 12 hours or more and, more preferably, 16-20 hours or more.

Dosage forms described herein exhibit uniform release rates of oxycodone for a prolonged period of time within the sustained release time period.

By "uniform release rate" is meant an average hourly release rate from the core that varies positively or negatively by no more than about 30% and preferably no more than about 25% and most preferably no more than 10% from either the preceding or the subsequent average hourly release rate as determined in a USP Type 7 Interval Release Apparatus where the cumulative release is between about 25% to about 75%.

By "prolonged period of time" is meant a continuous period of time of at least about 4 hours, preferably 6-8 hours or more and, more preferably, 10 hours or more. For example, the exemplary osmotic dosage forms described herein generally begin releasing oxycodone at a uniform release rate within about 2 to about 6 hours following administration and the uniform rate of release, as defined above, continues for a prolonged period of time from about 25% to until at least about 75% and preferably at least about 85% of the drug is released from the dosage form. Release of oxycodone continues thereafter for several more hours although the rate of release is generally slowed somewhat from the uniform release rate.

By "C" is meant the concentration of drug in the blood plasma of a subject, generally expressed as mass per unit volume, typically nanograms per milliliter. For convenience, this concentration may be referred to as "plasma drug concentration" or "plasma concentration" herein which is intended to be inclusive of drug concentration measured in any appropriate body fluid or tissue. The plasma drug concentration at any time following drug administration is referenced as Cₜᵢₘₑ, as in C₉ₕ or C₂₄ₕ, etc.

By "steady state" is meant the condition in which the amount of drug present in the blood plasma of a subject does not vary significantly over a prolonged period of time. A pattern of drug accumulation following continuous administration of a constant dose and dosage form at constant dosing intervals eventually achieves a "steady-state" where the plasma concentration peaks and plasma concentration troughs are essentially identical within each dosing interval. As used herein, the steady state maximal (peak) plasma drug concentration is referenced as Cₘₐₓ and the minimal (trough) plasma drug concentration is referenced as Cₘᵢₙ. The times following drug administration at which the steady-state peak plasma and trough drug concentrations occur are referenced as the Tₘₐₓ and the Tₘᵢₙ, respectively.

Persons of skill in the art appreciate that plasma drug concentrations obtained in individual subjects will vary due to intrapatient variability in the many parameters affecting drug absorption, distribution, metabolism and excretion. For this reason, unless otherwise indicated, mean values obtained from groups of subjects are used herein for purposes of comparing plasma drug concentration data and for analyzing relationships between *in vitro* dosage form dissolution rates and *in vivo* plasma drug concentrations.

A relationship between an administered dose of oxycodone and the magnitude of the peak plasma oxycodone concentration obtained following dose administration is used herein to illustrate significant differences between the dosage forms and methods described herein and prior art dosage forms. For example, as described below in more detail, a unitless numerical value is derived by calculating the ratio of the numerical value of the mean Cₘₐₓ (ng/ml) to the numerical value of the dose (mg), i.e., Cₘₐₓ/dose. The difference in the values of the derived ratios characterize the reduction in the magnitude of peak plasma oxycodone concentrations following administration, of the sustained release oxycodone dosage forms described herein compared to peak plasma oxycodone concentrations following administration of conventional immediate-release oxycodone dosage forms. Administration of dosage forms described herein preferably provides steady-state Cₘₐₓ/dose ratios of less than about 30 and more preferably less than about 25.

It has been surprisingly discovered that sustained release oxycodone dosage forms exhibiting T₇₀ values of about 10 to 20 hours and preferably 15 to 18 hours and more preferably at about 17 hours or more which release oxycodone at a uniform release rate fort prolonged period of time can be prepared. Administration of such dosage forms once daily provides therapeutically effective average steady-state plasma oxycodone concentrations.

The exemplary sustained release oxycodone dosage forms, methods of preparing such dosage forms and methods of using such dosage forms described herein are directed to osmotic dosage forms for oral administration. In addition to osmotic systems as described herein, however, there are many other approaches to achieving sustained release of drugs from oral dosage forms known in the art. These different approaches may include, for example, diffusion systems such as reservoir devices and matrix devices, dissolution systems such as encapsulated dissolution systems (including, for example, "tiny time pills") and matrix dissolution systems, combination diffusion/dissolution systems and ion-exchange resin systems as described in Remington's Pharmaceutical Sciences. 1990 ed., pp. 1682-1685. The use of oxycodone in the manufacture of dosage forms that operate in accord with these other approaches is encompassed by the scope of the claims below to the extent that the drug release characteristics and/or the plasma oxycodone concentration characteristics as recited in the claims describe those dosage forms either literally or equivalently.

Osmotic dosage forms, in general, utilized osmotic pressure to generate a driving force for imbibing fluid into a compartment formed, at least in part, by a semipermeable wall that permits free diffusion of fluid but not drug or osmotic agent(s), if present A significant advantage to osmotic systems is that operation is pH-independent and thus continues at the osmotically determined rate throughout an extended time period even as the dosage form transits the gastrointestinal tract and encounters differing microenvironments having significantly different pH values. A review of such dosage forms is found in Santus and Baker, "Osmotic drug delivery: a review of the patent literature," Journal of Controlled Release 35 (1995) 1-21.
In particular, the following U.S. Patents, owned by the assignee of the present application, ALZA Corporation, are directed to osmotic dosage forms;
Nos. 3,845,770; 3,916,899; 3,995,531; 4,008,719; 4,111,202; 4,160,020; 4,327,725; 4,519,801; 4,578,075; 4,681,583; 5,019,397; and 5,156,850.

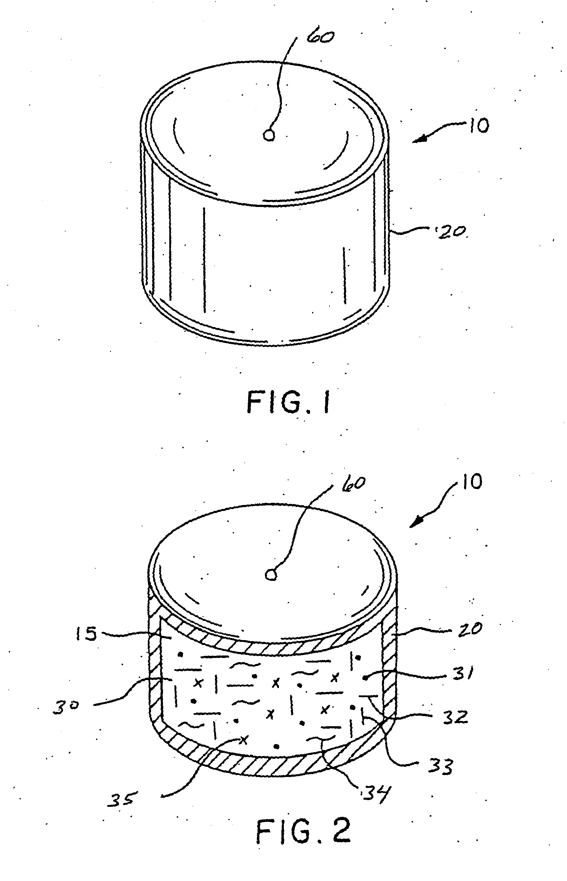

Figure 1 is perspective view of one example of a sustained release osmotic dosage form.
Dosage form 10 comprises wall 20 that surrounds and encloses an internal compartment (not seen in Figure 1). The internal compartment contains a composition comprising oxycodone, or a pharmaceutically acceptable and addition salt thereof, as described in more detail below. Wall 20 is provided with at least one drug delivery exit 60 for connecting the internal compartment with the exterior environment of use. Accordingly, following oral ingestion of dosage form 10, fluid is imbibed through wall 20 and oxycodone is released through exit 60. While the preferred geometrical example in Figure 1 illustrates a standard biconvex shaped tablet, the geometry may embrace a capsule shaped caplet and other oral, buccal, or sublingual dosage forms.

It has been discovered that such dosage forms provide improved compliance and convenience as well as a reduction in side effects associated with administration of oxycodone, increased tolerance, enhanced efficacy. It has been further discovered that additional indications are responsive to the administration of the dosage form.

Figure 2 is a cutaway view of Figure 1 showing an example of a dosage form with internal compartment 15 containing a single component layer referred to herein as drug layer 30, comprising oxycodone drug 31 in an admixture with selected excipients adapted to provide an osmotic activity gradient for driving fluid from an external environment through wall 20 and for forming a deliverable oxycodone formulation upon imbibition of fluid. As described in more detail below, the excipients may include a suitable suspending agent, also referred to herein as drug carrier 32, binder 33, lubricant 34 and an osmotically active agent, osmagent 35. In operation, following oral ingestion of dosage form 10, the osmotic activity gradient across wall 20 causes gastric fluid to be imbibed through the wall 20 thereby forming a deliverable oxycodone formulation, i.e., a solution or suspension, within the internal compartment. The deliverable oxycodone formulation is released through exit 60 as fluid continues to enter the internal compartment. As release of drug formulation occurs, fluid continues to be imbibed thereby driving continued release. In this manner, oxycodone is released in a sustained and continuous manner over an extended time period.

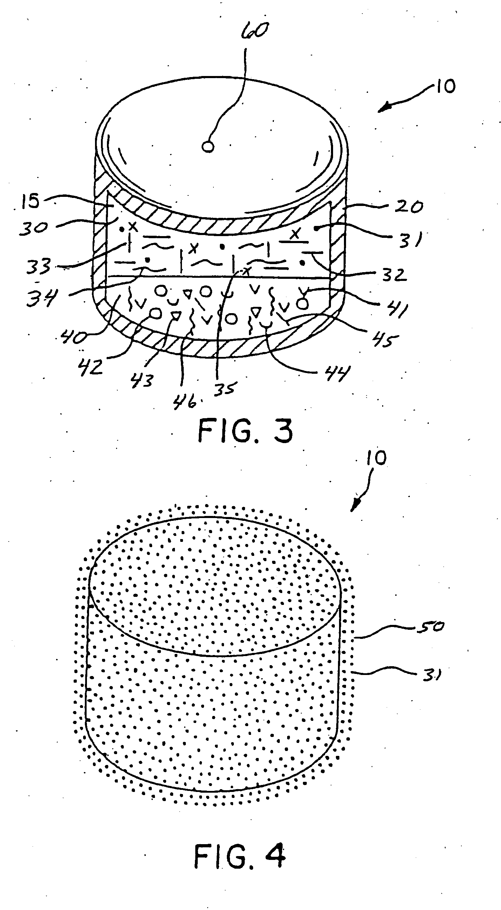

Figure 3 is a cutaway view of Figure 1 with an alternate example of internal compartment 15 having a bilayer configuration. In this example, internal compartment 15 contains a bilayered-compressed core having a first component drug layer 30 and a second component push layer 40. Drug layer 30, as described above with reference to Figure 1, comprises oxycodone in an admixture with selected excipients.

As described in more detail below, second component push layer 40 comprises osmotically active component(s), but does not contain any active agent. The components in push layer 40 typically comprise an osmagent 42 and one or more osmopolymer 41 having relatively large molecular weights which exhibit swelling as fluid is imbibed such that release of these osmopolymers through the drug delivery orifice 60 does not occur. Additional excipients such as binder 43, lubricant 44, antioxidant 45 and colorant 46 may also be included in push layer 40. The second component layer is referred to herein as an expandable or a push layer since, as fluid is imbibed, the osmopolymer(s) swell and push against the deliverable drug formulation of the first component drug layer to thereby facilitate release of the drug formulation from the dosage form.

In operation, following oral ingestion of the dosage form 10 as shown in Figure 3, the osmotic activity gradient across wall 20 causes gastric fluid to be imbibed through wall 20 thereby forming drug layer 30 into a deliverable formulation and concurrently swelling the osmopolymer(s) in push layer 40. The deliverable drug layer 30 is released through exit 60 as fluid continues to enter internal compartment 15 and push layer 40 continues to swell. As release of drug layer 30 occurs, fluid continues to be imbibed and the push layer continues to swell thereby driving continued release. In this manner, oxycodone is released in a sustained and continuous manner over an extended time period.

Drug layer 30, as described with reference to Figures 2 and 3, comprises oxycodone in an admixture with selected excipients. Push layer 40, as described with reference to Figure 3, comprises osmotically active component(s) but does not contain any active agent.

Drug layer 30 comprises a composition formed of a pharmaceutically effective amount of oxycodone drug 31, or a pharmaceutically acceptable salt thereof, and a carrier 32. The drug oxycodone is comprised of 4, 5-Epoxy-14-hydroxy-3-methoxy17-methylmorphinian-6-one possessing analygesic therapy. Oxycodone is known in the art. The Merck Index, 11th Ed., p. 1100 (1990). The oxycodone salts are represented by a member selected from the group consisting of the following: oxycodone sulfate, oxycodone hydrochloride, oxycodone trifluoracetate, oxycodone thiosemicarbazone hydrochloride, oxycodone pentafluaropropionate, oxycodone p-nitrophenylhydrozone, oxycodone o-methyloxine, oxycodone thiosemicarbazone, oxycodone semicarbazone, oxycodone phenylhydroazone, oxycodone hydrazone, oxycodone hydrobromide, oxycodone mucate, oxycodone methylbromide, oxycodone oleate, oxycodone n-oxide, oxycodone acetate, oxycodone phosphate dibasic, oxycodone phosphate monobasic, oxycodone inorganic salt, oxycodone organic salt, oxycodone acetate trihydrate, oxycodone bis(heptafluorobutyrate), oxycodone bis(methylcarbamate), oxycodone (bis-pentafluoropropionate), oxycodone bis(pyridine-3-carboxylate), oxycodone bis(trifluoroacetate), oxycodone bitartrate, oxycodone chlorohydrate and oxycodone sulfate pentahydrate.

The dosage form and the therapeutic composition in either manufacture comprise 1 to 640 mg of oxycodone drug 31 or oxycodone drug 31 pharmaceutically acceptable salt. Preferably the dosage form described herein comprises 20 mg to 160 mg of oxycodone drug 31.

Carrier 32 may comprise a hydrophilic polymer represented by horizontal dashes in Figure 2 and Figure 3. The hydrophilic polymer provides a hydrophilic polymer particle in the drug composition that contributes to the controlled delivery of active agent Representative examples of these polymers are poly(alkylene oxide) of 100,000 to 750,000 number-average molecular weight, including poly(ethylene oxide), poly(methylene oxide), poly(butylene oxide) and poly(hexytene oxide); and a poly(carboxymethylcellulose) of 40,000 to 400,000 number-average molecular weight, represented by poly(alkali carboxymethylcellulose), poly(sodium carboxymethylcellulose), poly(potassium carboxymethylcellulose) and poly(lithium carboxymethylcellulose). The drug composition can comprise a hydroxypropylalkylcellulose of 9,200 to 125,000 number-average molecular weight for enhancing the delivery properties of the dosage form as represented by hydroxypropylethylcellulose, hydroxypropylmethylcellulose, hydroxypropylbutylcellulose and hydroxypropylpentylcellulose; and a poly(vinylpyrrolidone) of 7,000 to 75,000 number-average molecular weight for enhancing the flow properties of the dosage form. Preferred among those polymers are the poly(ethylene oxide) of 100,000 - 300,000 number average molecular weight. Carriers that erode in the gastric environment, i.e., bioerodible carriers, are especially preferred.

Other carriers that may be incorporated into drug layer 30 include carbohydrates that exhibit sufficient osmotic activity to be used alone or with other osmagents. Such carbohydrates comprise monsaccharides, disaccharides and polysaccharides. Representative examples include maltodextrins (i.e., glucose polymers produced by the hydrolysis of com starch) and the sugars comprising lactose, glucose, raffinose, sucrose, mannitol, sorbitol, and the like. Preferred maltodextrins are those having a dextrose equivalence (DE) of 20 or less, preferably with a DE ranging from about 4 to about 20, and often 9-20. Maltodextrin having a DE of 9-12 has been found most useful.

Carbohydrates described above, preferably the maltodextrins, may be used in the drug layer 30 without the addition of an osmagent, and obtain the desired release of oxycodone from the dosage form, while providing a therapeutic effect over a prolonged period of time and up to 24 hours with once-a-day dosing.

Drug layer 30 may further comprise a therapeutically acceptable vinyl polymer binder 33 represented by vertical dashes in Figure 2 and Figure 3. The vinyl polymer comprises a 5,000 to 350,000 average molecular weight, represented by a member selected from the group consisting of poly-n-vinylamide, poly-n-vinylacetamide, poly(vinyl pyrrolidone), also known as poly-n-vinylpyrrolidone, poly-n-vinylcaprolactone, poly-n-vinyl-5-methyl-2-pyrrolidone, and poly-n-vinylpyrrolidone copolymers with a member selected from the group consisting of vinyl acetate, vinyl alcohol, vinyl chloride, vinyl fluoride, vinyl butyrate, vinyl laureate, and vinyl stearate. Dosage form 10 and the therapeutic composition comprises 0.01 to 25 mg of the binder or vinyl polymer that serves as a binder. Representative of other binders include acacia, starch and gelatin.

Dosage form 30 may further comprise lubricant 34 represented by a wavy line in Figure 2 and Figure 3. The lubricant is used during manufacture to prevent sticking to die walls or punch faces. Typical lubricants include magnesium stearate, sodium stearate, stearic acid, calcium stearate, magnesium oleate, oleic acid, potassium oleate, caprylic acid, sodium stearyl fumarate, and magnesium palmitate. The amount of lubricant present in the therapeutic composition is 0.01 to 10 mg.

Drug layer 30 typically will be a dry composition formed by compression of the carrier and the drug as one layer and the push composition as the other layer in contacting relation.

Drug layer 30 is formed as a mixture containing oxycodone and the carrier that when contacted with biological fluids in the environment of use provides a slurry, solution or suspension of the compound that may be dispensed by the action of the push layer. The drug layer may be formed from particles by comminution that produces the size of the drug and the size of the accompanying polymer used in the fabrication of the drug layer, typically as a core containing the compound.
The means for producing particles include granulation, spray drying, sieving, lyophilization, crushing, grinding, jet milling, micronizing and chopping to produce the intended micron particle size. The process can be performed by size reduction equipment, such as a micropulverizer mill, a fluid energy grinding mill, a grinding mill, a roller mill, a hammer mill, an attrition mill, a chaser mill, a ball mill, a vibrating ball mill, an impact pulverizer mill, a centrifugal pulverizer, a coarse crusher and a fine crusher. The size of the particle can be ascertained by screening, including a grizzly screen, a flat screen, a vibrating screen, a revolving screen, a shaking screen, an oscillating screen and a reciprocating screen. The processes and equipment for preparing drug and carrier particles are disclosed in Pharmaceutical Sciences, Remington, 17th Ed., pp. 1585-1594 (1985); Chemical Engineers Handbook, Perry, 6th Ed., pp. 21-13 to 21-19 (1984); Journal of Pharmaceutical Sciences, Parrot, Vol. 61, No. 6, pp. 813-829 (1974); and Chemical Engineer, Hixon, pp. 94-103 (1990).

Drug layer 30 may further comprise surfactants and disintegrants. Exemplary of the surfactants are those having an HLB value of between about 10 - 25, such as polyethylene glycol 400 monostearate, polyoxyethylene-4-sorbitan monolaurate, polyoxyethylene-20-sorbitan monooleate, polyoxyethylene-20-sorbitan monopalmitate, polyoxyethylene-20-monolaurate, polyoxyethylene-40 -stearate, sodium oleate and the like. Disintegrants may be selected from starches, clays, celluloses, algins and gums and crosslinked starches, celluloses and polymers. Representative disintegrants include corn starch, potato starch, croscarmelose, crospovidone, sodium starch glycolate, Veegum HV, methylcellulose, agar, bentonite, carboxymethylcellulose, alginic acid, guar gum and the like.

The active agent may be provided in the drug layer in amounts of from 0.1 mg to 640 mg per dosage form, preferably 10 mg to 80 mg per dosage form, and more preferably 20 mg to 80 mg, depending upon the required dosing level that must be maintained over the delivery period, i.e., the time between consecutive administrations of the dosage forms. More typically, loading of compound in the dosage forms will provide doses of compound to the subject ranging from 10 mg to 160 mg and more usually 20 mg to 80 mg per day. Generally, if a total drug dose of more than 160 mg per day is required, multiple units of the dosage form may be administered at the same time to provide the required amount of drug.

As a representative compound of the compounds having pain relieving activity described herein, immediate release oxycodone is typically administered at a starting dose of about 10 mg, administered in two or three doses per day. The effective dose range has been determined to be generally 10 mg/day - 320 mg/day. Observation of tolerability and need for additional clinical effect over the starting dose often results in the dose being increased in increments of 5 mg/day to 80 mg/day.

Concurrently with observation, plasma concentrations in a subject may be determined by clinical assay to determine a correlation between tolerability and clinical effect and blood plasma concentrations of drug. Plasma concentrations may range from 0.1 ng/ml to 100 ng/ml (nanograms per milliliter), more typically 4 ng/ml to 40 ng/ml, of compound.

Push layer 40 comprises a displacement composition in contacting layered arrangement with the first component drug layer 30 as illustrated in Figure 3. Push layer 40 comprises osmopolymer 41 that imbibes an aqueous or biological fluid and swells to push the drug composition through the exit means of the device. A polymer having suitable imbibition properties may be referred to herein as an osmopolymer. The osmopolymers are swellable, hydrophilic polymers that interact with water and aqueous biological fluids and swell or expand to a high degree, typically exhibiting a 2-50 fold volume increase. The osmopolymer can be non-crosslinked or crosslinked, but in a referred example are at least lightly crosslinked to create a polymer network that is too large and entangled to exit the dosage form. Thus, in a preferred example, the expandable composition is retrained within the dosage form during its operative lifetime.

Push layer 40 comprises 20 to 375 mg of osmopolymer 41, represented by "V" in Figure 3. Osmopolymer 41 in layer 40 possesses a higher molecular weight than osmopolymer 32 in drug layer 20.

Representatives of fluid-imbibing displacement polymers comprise members selected from poly(alkylene oxide) of 1 million to 15 million number-average molecular weight, as represented by poly(ethylene oxide), and poly(alkali carboxymethylcellulose) of 500,000 to 3,500,000 number-average molecular weight, wherein the alkali is sodium, potassium or lithium. Examples of additional polymers for the formulation of the push-displacement composition comprise osmopolymers comprising polymers that form hydrogels, such as Carbopol^{®} acidic carboxypolymer, a polymer of acrylic cross-linked with a polyallyl sucrose, also known as carboxypolymethylene, and carboxyvinyl polymer having a molecular weight of 250,000 to 4,000,000; Cyanamer^{®} polyacrylamides; cross-linked water swellable indenemaleic anhydride polymers; Good-rite^{®} polyacrylic acid having a molecular weight of 80,000 to 200,000; Aqua-Keeps^{®} acrylate polymer polysaccharides composed of condensed glucose units, such as diester cross-linked polygluran; and the like. Representative polymers that form hydrogels are known to the prior art in U.S. Patent No. 3,865,108, issued to Hartop; U.S. Patent No. 4,002,173, issued to Manning; U.S. Patent No. 4,207,893, issued to Michaels; and in Handbook of Common Polymers, Scott and Roff, Chemical Rubber Co., Cleveland, OH.

Push layer 40 comprises 0 to 75 mg, and presently 5 to 75 mg of an osmotically effective compound, osmagent 42, represented by circles in Figure 3. The osmotically effective compounds are known also as osmagents and as osmotically effective solutes. Osmagent 42 that may be found in the drug layer and the push layer in the dosage form are those which exhibit an osmotic activity gradient across the wall 20. Suitable osmagents comprise a member selected from the group consisting of sodium chloride, potassium chloride, lithium chloride, magnesium sulfate, magnesium chloride, potassium sulfate, sodium sulfate, lithium sulfate, potassium acid phosphate, mannitol, urea, inositol, magnesium succinate, tartaric acid, raffinose, sucrose, glucose, lactose, sorbitol, inorganic salts, organic salts and carbohydrates.

Push layer 40 may further comprises a therapeutically acceptable vinyl polymer 43 represented by triangles in Figure 3. The vinyl polymer comprises a 5,000 to 350,000 viscosity-average molecular weight, represented by a member selected from the group consisting of poly-n-vinylamide, poly-n-vinylacetamide, poly(vinyl pyrrolidone), also known as poly-n-vinylpyrrolidone, poly-n-vinylcaprolactone, poly-n-vinyl-5-methyl-2-pyrrolidone, and poly-n-vinylpyrrolidone copolymers with a member selected from the group consisting of vinyl acetate, vinyl alcohol, vinyl chloride, vinyl fluoride, vinyl butyrate, vinyl laureate, and vinyl stearate. Push layer contains 0.01 to 25 mg of vinyl polymer.

Push layer 40 may further comprise 0 to 5 mg of a nontoxic colorant or dye 46, identified by vertical wavy lines in Figure 3. Colorant 35 includes Food and Drug Administration Colorant (FD&C), such as FD&C No. 1 blue dye, FD&C No. 4 red dye, red ferric oxide, yellow ferric oxide, titanium dioxide, carbon black, and indigo.

Push layer 40 may further comprise lubricant 44, identified by half circles in Figure 3. Typical lubricants comprise a member selected from the group consisting of sodium stearate, potassium stearate, magnesium stearate, stearic acid, calcium stearate, sodium oleate, calcium palmitate, sodium laurate, sodium ricinoleate and potassium linoleate. The concentration of lubricant is 0.01 to 10 mg.

Push layer 40 may further comprise an antioxidant 45. represented by slanted dashes in Figure 3 to inhibit the oxidation of ingredients comprising expandable formulation 40. Push layer 40 comprises 0.00 to 5 mg of an antioxidant. Representative antioxidants comprise a member selected from the group consisting of ascorbic acid, ascorbyl palmitate, butylated hydroxyanisole, a mixture of 2 and 3 tertiary-butyl-4-hydroxyanisole, butylated hydroxytoluene, sodium isoascorbate, dihydroguaretic acid, potassium sorbate, sodium bisulfate, sodium metabisulfate, sorbic acid, potassium ascorbate, vitamin E, 4-chloro-2,6-ditertiary butylphenol, alpha-tocopherol, and propylgallate.

Figure 4 depicts the preferred example comprising an overcoat 50 of drug 31 on the dosage form of Figure 3. Dosage form 10 of Figure 4 comprises an overcoat 50 on the outer surface of wall 20 of dosage form 10. Overcoat 50 is a therapeutic composition comprising 0.5 to 75 mg of oxycodone 31 and 0.5 to 275 mg of a pharmaceutically acceptable carrier selected from the group consisting of alkylcellulose, hydroxyalkylcellulose and hydroxypropylalkylcellulose. The overcoat is represented by methylcellulose, hydroxyethylcellulose, hydroxybutylcellulose, hydroxypropylcellulose, hydroxypropylmethylcellulose, hydroxypropytethylcellulose and hydroxypropylbutylcellulose. Overcoat 50 provides therapy immediately as overcoat 50 dissolves or undergoes dissolution in the presence of gastrointestinal fluid and concurrently therewith delivers oxycodone drug 31 into the gastrointestinal tract for immediate oxycodone therapy.

Exemplary solvents suitable for manufacturing the dosage form components comprise aqueous or inert organic solvents that do not adversely harm the materials used in the system. The solvents broadly include members selected from the group consisting of aqueous solvents, alcohols, ketones, esters, ethers, aliphatic hydrocarbons, halogenated solvents, cycloaliphatics, aromatics, heterocyclic solvents and mixtures thereof. Typical solvents include acetone, diacetone alcohol, methanol, ethanol, isopropyl alcohol, butyl alcohol, methyl acetate, ethyl acetate, isopropyl acetate, n-butyl acetate, methyl isobutyl ketone, methyl propyl ketone, n-hexane, n-heptane, ethylene glycol monoethyl ether, ethylene glycol monoethyl acetate, methylene dichloride, ethylene dichloride, propylene dichloride, carbon tetrachloride nitroethane, nitropropane tetrachloroethane, ethyl ether, isopropyl ether, cyclohexane, cyclooctane, benzene, toluene, naphtha, 1,4-dioxane, tetrahydrofuran, diglyme, water, aqueous solvents containing inorganic salts such as sodium chloride, calcium chloride, and the like, and mixtures thereof such as acetone and water, acetone and methanol, acetone and ethyl alcohol, methylene dichloride and methanol, and ethylene dichloride and methanol.

Wall 20 is formed to be permeable to the passage of an external fluid, such as water and biological fluids, and it is substantially impermeable to the passage of oxycodone, osmagent, osmopolymer and the like. As such, it is semipermeable. The selectively semipermeable compositions used for forming the wall are essentially nonerodible and they are substantially insoluble in biological fluids during the life of the dosage form.

Representative polymers for forming wall 20 comprise semipermeable homopolymers, semipermeable copolymers, and the like. Such materials comprise cellulose esters, cellulose ethers and cellulose ester-ethers. The cellulosic polymers have a degree of substitution (DS) of their anhydroglucose unit of from greater than 0 up to 3, inclusive. Degree of substitution (DS) means the average number of hydroxyl groups originally present on the anhydroglucose unit that are replaced by a substituting group or converted into another group. The anhydroglucose unit can be partially or completely substituted with groups such as acyl, alkanoyl, alkenoyl, aroyl, alkyl, alkoxy, halogen, carboalkyl, alkylcarbamate, alkylcarbonate, alkylsulfonate, alkysulfamate, semipermeable polymer forming groups, and the like, wherein the organic moieties contain from one to twelve carbon atoms, and preferably from one to eight carbon atoms.

The semipermeable compositions typically include a member selected from the group consisting of cellulose acylate, cellulose diacylate, cellulose triacylate, cellulose acetate, cellulose diacetate, cellulose triacetate, mono-, di- and tri-cellulose alkanylates, mono-, di-, and tri-alkenylates, mono-, di-, and tri-aroylates, and the like. Exemplary polymers include cellulose acetate having a DS of 1.8 to 2.3 and an acetyl content of 32 to 39.9%; cellulose diacetate having a DS of 1 to 2 and an acetyl content of 21 to 35%; cellulose triacetate having a DS of 2 to 3 and an acetyl content of 34 to 44.8%; and the like. More specific cellulosic polymers include cellulose propionate having a DS of 1.8 and a propionyl content of 38.5%; cellulose acetate propionate having an acetyl content of 1.5 to 7% and an acetyl content of 39 to 42%; cellulose acetate propionate having an acetyl content of 2.5 to 3%, an average propionyl content of 39.2 to 45%, and a hydroxyl content of 2.8 to 5.4%; cellulose acetate butyrate having a DS of 1.8, an acetyl content of 13 to 15%, and a butyryl content of 34 to 39%; cellulose acetate butyrate having an acetyl content of 2 to 29%, a butyryl content of 17 to 53%, and a hydroxyl content of 0.5 to 4.7%; cellulose triacylates having a DS of 2.6 to 3, such as cellulose trivalerate, cellulose trilamate, cellulose tripalmitate, cellulose trioctanoate and cellulose tripropionate; cellulose diesters having a DS of 2.2 to 2.6, such as cellulose disuccinate, cellulose dipalmitate, cellulose dioctanoate, cellulose dicaprylate, and the like; and mixed cellulose esters, such as cellulose acetate valerate, cellulose acetate succinate, cellulose propionate succinate, cellulose acetate octanoate, cellulose valerate palmitate, cellulose acetate heptanoate, and the like. Semipermeable polymers are known in U.S. Patent No. 4,077,407, and they can be synthesized by procedures described in Encyclopedia of Polymer Science and Technology, Vol. 3, pp. 325-354 (1964), Interscience Publishers Inc., New York, NY.

Additional semipermeable polymers for forming the outer wall 20 comprise cellulose acetaldehyde dimethyl acetate; cellulose acetate ethylcarbamate; cellulose acetate methyl carbamate; cellulose dimethylaminoacetate; semipermeable polyamide; semipermeable polyurethanes; semipermeable sulfonated polystyrenes; cross-linked selectively semipermeable polymers formed by the coprecipitation of an anion and a cation, as disclosed in U.S. Patents Nos. 3,173,876; 3,276,586; 3,541,005; 3,541,006 and 3,546,142; semipermeable polymers, as disclosed by Loeb, et al. in U.S. Patent No. 3,133,132; semipermeable polystyrene derivatives; semipermeable poly(sodium styrenesulfonate); semipermeable poly(vinylbenzyltrimethylammonium chloride); and semipermeable polymers exhibiting a fluid permeability of 10⁻⁵ to 10⁻² (cc. mil/cm hr.atm), expressed as per atmosphere of hydrostatic or osmotic pressure differences across a semipermeable wall. The polymers are known to the art in U.S. Patents Nos. 3,845,770; 3,916,899 and 4,160,020; and in Handbook of Common Polymers, Scott and Roff (1971) CRC Press, Cleveland, OH.

Wall 20 may also comprise a flux-regulating agent. The flux regulating agent is a compound added to assist in regulating the fluid permeability or flux through wall 20. The flux-regulating agent can be a flux-enhancing agent or a flux-decreasing agent. The agent can be preselected to increase or decrease the liquid flux. Agents that produce a marked increase in permeability to fluid such as water are often essentially hydrophilic, while those that produce a marked decrease to fluids such as water are essentially hydrophobic. The amount of regulator in the wall when incorporated therein generally is from about 0.01% to 20% by weight or more. The flux regulator agents may include polyhydric alcohols, polyalkylene glycols, polyalkylenediols, polyesters of alkylene glycols, and the like. Typical flux enhancers include polyethylene glycol 300, 400, 600, 1500, 4000, 6000 and the like; low molecular weight glycols such as polypropylene glycol, polybutylene glycol and polyamylene glycol: the polyalkylenediols such as poly(1,3-propanediol), poly(1,4-butanediol), poly(1 ,6-hexanediol), and the like; aliphatic diols such as 1,3-butylene glycol, 1,4-pentamethylene glycol, 1,4-hexamethylene glycol, and the like; alkylene triols such as glycerine, 1,2,3-butanetriol, 1,2,4-hexanetriol, 1,3,6-hexanetriol and the like; esters such as ethylene glycol dipropionate, ethylene glycol butyrate, butylene glycol dipropionate, glycerol acetate esters, and the like. Presently preferred flux enhancers include the group of difunctional block-copolymer polyoxyalkylene derivatives of propylene glycol known as pluronics (BASF). Representative flux-decreasing agents include phthalates substituted with an alkyl or alkoxy or with both an alkyl and alkoxy group such as diethyl phthalate, dimethoxyethyl phthalate, dimethyl phthalate, and [di(2-ethylhexyl) phthalate], aryl phthalates such as triphenyl phthalate, and butyl benzyl phthalate; polyvinyl acetates, triethyl citrate, eudragit; insoluble salts such as calcium sulfate, barium sulfate, calcium phosphate, and the like; insoluble oxides such as titanium oxide; polymers in powder, granule and like form such as polystyrene, polymethylmethacrylate, polycarbonate, and polysulfone; esters such as citric acid esters esterified with long chain alkyl groups; inert and substantially water impermeable fillers; resins compatible with cellulose based wall forming materials, and the like.

Other materials may be included in the semipermeable wall material for imparting flexibility and elongation properties, to make wall 20 less brittle and to render tear strength. Suitable materials include phthalate plasticizers such as dibenzyl phthalate, dihexyl phthalate, butyl octyl phthalate, straight chain phthalates of six to eleven carbons, di-isononyl phthalte, di-isodecyl phthalate, and the like. The plasticizers include nonphthalates such as triacetin, dioctyl azelate, epoxidized tallate, tri-isoctyl trimellitate, tri-isononyl trimellitate, sucrose acetate isobutyrate, epoxidized soybean oil, and the like. The amount of plasticizer in a wall when incorporated therein is about 0.01 % to 20% weight, or higher.

Pan coating may be conveniently used to provide the completed dosage form, except for the exit orifice. In the pan coating system, the wall-forming composition for wall 20 is deposited by successive spraying of the appropriate wall composition onto the compressed single or bilayered core comprising the drug layer for the single layer core or the drug layer and the push layer for the bilayered core, accompanied by tumbling in a rotating pan. A pan coater is used because of its availability at commercial scale. Other techniques can be used for coating the compressed core. Once coated, the wall is dried in a forced-air oven or in a temperature and humidity controlled oven to free the dosage form of solvent(s) used in the manufacturing. Drying conditions will be conventionally chosen on the basis of available equipment, ambient conditions, solvents, coatings, coating thickness, and the like.

Other coating techniques can also be employed. For example, the wall or walls of the dosage form may be formed in one technique using the air-suspension procedure. This procedure consists of suspending and tumbling the compressed single or bilayer core in a current of air and the semipermeable wall forming composition, until the wall is applied to the core. The air-suspension procedure is well suited for independently forming the wall of the dosage form. The air-suspension procedure is described in U.S. Patent No. 2,799,241; in J. Am. Pharm. Assoc.. Vol. 48, pp. 451-459 (1959); and, ibid., Vol. 49, pp. 82-84 (1960). The dosage form also can be coated with a Wurster^{®} air-suspension coater using, for example, methylene bichloride methanol as a cosolvent for the wall forming material. An Aeromatic^{®} air-suspension coater can be used employing a cosolvent.

Dosage forms are manufactured by standard techniques. For example, the dosage form may be manufactured by the wet granulation technique. In the wet granulation technique, the drug and carrier are blended using an organic solvent, such as denatured anhydrous ethanol, as the granulation fluid. The remaining ingredients can be dissolved in a portion of the granulation fluid, such as the solvent described above, and this latter prepared solution is slowly added to the drug blend with continual mixing in the blender. The granulating fluid is added until a wet blend is produced, which wet mass blend is then forced through a predetermined screen onto oven trays. The blend is dried for 18 to 24 hours at 24°C to 35°C in a forced-air oven. The dried granules are then sized. Next, magnesium stearate, or another suitable lubricant, is added to the drug granulation, and the granulation is put into milling jars and mixed on a jar mill for 10 minutes. The composition is pressed into a layer, for example, in a Manesty^{®} press or a Korsch LCT press. For a bilayered core, the drug-containing layer is pressed and a similarly prepared wet blend of the push layer composition, if included, is pressed against the drug-containing layer. The intermediate compression typically takes place under a force of about 50-100 newtons. Final stage compression typically takes place at a force of 3500 newtons or greater, often 3500-5000 newtons. The single or bilayer compressed cores are fed to a dry coater press, e.g., Kilian^{®} Dry Coater press, and subsequently coated with the wall materials as described above.

One or more exit orifices are drilled in the drug layer end of the dosage form, and optional water soluble overcoats, which may be colored (e.g., Opadry colored coatings) or clear (e.g., Opadry Clear), may be coated on the dosage form to provide the finished dosage form.

In another manufacture the drug and other ingredients comprising the drug layer are blended and pressed into a solid layer. The layer possesses dimensions that correspond to the internal dimensions of the area the layer is to occupy in the dosage form, and it also possesses dimensions corresponding to the second push layer, if included, for forming a contacting arrangement therewith. The drug and other ingredients can also be blended with a solvent and mixed into a solid or semisolid form by conventional methods, such as ballmilling, calendering, stirring or rollmilling, and then pressed into a preselected shape. Next, if included, a layer of osmopolymer composition is placed in contact with the layer of drug in a like manner. The layering of the drug formulation and the osmopolymer layer can be fabricated by conventional two-layer press techniques. The compressed cores then may be coated with the semipermeable wall material as described above.

Another manufacturing process that can be used comprises blending the powdered ingredients for each layer in a fluid bed granulator. After the powdered ingredients are dry blended in the granulator, a granulating fluid, for example, poly(vinylpyrrolidone) in water, is sprayed onto the powders. The coated powders are then dried in the granulator. This process granulates all the ingredients present therein while adding the granulating fluid. After the granules are dried, a lubricant, such as stearic acid or magnesium stearate, is mixed into the granulation using a blender e.g., V-blender or tote blender. The granules are then pressed in the manner described above.

Exit 60 is provided in each dosage form. Exit 60 cooperates with the compressed core for the uniform release of drug from the dosage form. The exit can be provided during the manufacture of the dosage form or during drug delivery by the dosage form in a fluid environment of use.

Exit 60 may include an orifice that is formed or formable from a substance or polymer that erodes, dissolves or is leached from the outer wall to thereby form an exit orifice. The substance or polymer may include, for example, an erodible poly(glycolic) acid or poly(lactic) acid in the semipermeable wall; a gelatinous filament; a water-removable poly(vinyl alcohol); a leachable compound, such as a fluid removable pore-former selected from the group consisting of inorganic and organic salt, oxide and carbohydrate.

The exit, or a plurality of exits, can be formed by leaching a member selected from the group consisting of sorbitol, lactose, fructose, glucose, mannose, galactose, talose, sodium chloride, potassium chloride, sodium citrate and mannitol to provide a uniform-release dimensioned pore-exit orifice.

The exit can have any shape, such as round, triangular, square, elliptical and the like for the uniform metered dose release of a drug from the dosage form.

The dosage form can be constructed with one or more exits in spaced-apart relation or one or more surfaces of the dosage form.

Drilling, including mechanical and laser drilling, through the semipermeable wall can be used to form the exit orifice. Such exits and equipment for forming such exits are disclosed in U.S. Patents Nos. 3,916,899, by Theeuwes and Higuchi and in U.S. Patent No. 4,088,864, by Theeuwes, et al.
It is presently preferred to utilize a single exit orifice.
The unique release rate profile described herein provides efficacious oxycodone therapy over 24 hours. This dosage form releases oxycodone for about 24 hours after administration with an immediate release drug overcoat delivery and controlled drug delivery continuing thereafter until the core ceases to release drug.

The release rate is characterized by a T₇₀ of about 10 to 20 hours and preferably 15 to 18 hours and more preferably about 17 hours. The dosage form is further characterized by having Cₘₐₓ occur at great than 15 hours after administration and be less than twice C₂₄ to create a flatter blood plasma concentration profile over 24 hours. The profile is remarkable in that even with an immediate release coating, and its concomitant peak plasma concentration, the maximum blood plasma concentration does not occur until about 15 hours after administration. This novel profile provides efficacious therapy while maintaining drug plasma levels low enough to reduce side effects associated with high blood plasma concentration levels. This delivery profile also provides 24 hours of efficacy without high plasma levels and without sub-therapeutic blood levels.

In accord with the above-cited information obtained through experience with the conventional immediate-release dosage form, oxycodone may be provided in the drug layer In the sustained release dosage forms in amounts of about 10 mg to up to 100 mg or more, if desired. In presently preferred single drug layer examples of once-a-day dosage forms, the drug layer comprises oxycodone in a dose of 20 mg to 80 mg oxycodone per dosage form.

Representative dosage forms had T₇₀ values of greater than 14 hours and released oxycodone for a continuous period of time of more than about 22 hours. Within about 2 hours following administration, each of the different dosage forms were releasing oxycodone from the core at a uniform release rate that continued for a prolonged period of time of about 22 hours or more. This release in the referred example occurred subsequent to release of the immediate release coating.

In a bilayer example of once-a-day dosage forms the dosage forms have a T₇₀ of about 15 to 18 hours and preferably about 17 hours and provided release of oxycodone for a continuous period of time of at least about 24 hours. Within about 2 hours following administration, oxycodone is being released at a uniform release rate that continues for a prolonged period of time. Following this prolonged period of uniform release rates, drug release continues for several more hours until the dosage form is spent.

Dosage forms exhibit sustained release of drug over a continuous time period that includes a prolonged time when drug is released at a uniform release rate as determined in a standard release rate assay such as that described herein. When administered to a subject, the dosage forms provide blood plasma drug concentrations in the subject that are less variable over a prolonged period of time than those obtained with immediate release dosage forms. When the dosage forms are administered on a continuous once-a-day basis, the dosage forms provide therapeutically effective average steady-state plasma oxycodone concentrations while providing steady-state peak plasma oxycodone concentrations that occur at a later time following dose administration and that exhibit a lesser magnitude than the steady-state peak plasma oxycodone concentrations that occur following administration of immediate-release oxycodone dosage forms and existing extended release dosage forms.

Described herein is a method of treating disease states and conditions that are responsive to treatment with oxycodone by orally administering to a subject a sustained release dosage form of oxycodone. The method is practiced with dosage forms that are adapted to release the compound at a uniform release rate of between about 1 %/hr to about 6%/hr over a prolonged time period of at least about 20 hours, preferably 22 hours or more.

The practice of the foregoing methods by orally administering a oxycodone dosage form to a subject once a day for the treatment of pain is preferred. Other disease states and conditions, which may be manifested or clinically diagnosed as symptoms of pain, may be treated with the oxycodone dosage forms and methods described herein. In addition, other disease states and conditions which may or may not manifest in association with pain but which may be responsive to treatment with oxycodone may also be treated with the dosage forms and methods described herein.

Preferred methods of manufacturing dosage forms are generally described in the examples below. All percentages are weight percent unless otherwise noted.

### EXAMPLES 1

### Oxycodone Hydrochloride Biconvex Shaped Bilayer 20 mg System

A dosage form adapted, designed and shaped as an osmotic drug delivery device is manufactured as follows: 1933 g of oxycodone hydrochloride, USP, 7803 g of polyethylene oxide with average molecular weight of 200,000, and 200 g of polyvinylpyrrolidone identified as K29-32 having an average molecular weight of 40,000 are added to a fluid bed granulator bowl. Next a binder solution is prepared by dissolving 500 g of the same polyvinylpyrrolidone in 4500 g of water. The dry materials are fluid bed granulated by spraying with 2000 g of binder solution. Next, the wet granulation is dried in the granulator to an acceptable moisture content, and sized using by passing through a 7-mesh screen. Next, the granulation is transferred to a blender and mixed with 2 g of butylated hydroxytoluene as an antioxidant and lubricated with 25 g of magnesium stearate.

Next, a push composition is prepared as follows: first, a binder solution is prepared. 15.6 kg of polyvinylpyrrolidone identified as K29-32 having an average molecular weight of 40,000 is dissolved in 104.4 kg of water. Then, 24 kg of sodium chloride and 1.2 kg of ferric oxide are sized using a Quadro Comil with a 21-mesh screen. Then, the screened materials and 88.44 kg of Polyethylene oxide (approximately 2,000,000 molecular weight) are added to a fluid bed granulator bowl. The dry materials are fluidized and mixed while 46.2 kg of binder solution is sprayed from 3 nuzzles onto the powder. The granulation is dried in the fluid-bed chamber to an acceptable moisture level. The coated granules are sized using a Fluid Air mill with a 7-mesh screen. The granulation is transferred to a tote tumbler, mixed with 15 g of butylated hydroxytoluene and lubricated with 294 g magnesium stearate.

Next, the oxycodone hydrochloride drug composition and the push composition are compressed into bilayer tablets. First, 113 mg of the oxycodone hydrochloride composition is added to the die cavity and pre-compressed, then, 103 mg of the push composition is added and the layers are pressed into a 5/16" diameter round, standard concave, bilayer arrangement.

The bilayered arrangements are coated with a semi-permeable wall. The wall forming composition comprises 99% cellulose acetate having a 39.8% acetyl content and 1% polyethylene glycol comprising a 3.350 viscosity-average molecular weight. The wall-forming composition is dissolved in an acetone:water (95:5 wt:wt) co solvent to make a 5% solids solution. The wall-forming composition is sprayed onto and around the bilayered arrangements in a pan coater until approximately 39 mg of membrane is applied to each tablet.

Next, one 40 mil (1 mm) exit passageway is laser drilled through the semi-permeable wall to connect the drug layer with the exterior of the dosage system. The residual solvent is removed by drying for 48 hours as 45 C. and 45% humidity. After drilling, the osmotic systems are dried for 4 hours at 45 C. to remove excess moisture.

Next, the drilled and dried systems are coated with an immediate release drug overcoat. The drug overcoat is a 8% solids aqueous solution containing 157.5 g of oxycodone HCl, USP and 850 g of hydroxypropyl methylcellulose possessing an average molecular weight of 11,200. The drug overcoat solution is sprayed onto the dried coated cores until an average wet coated weight of approximately 8 mg per system is achieved.

Next, the drug-overcoated systems are color overcoated. The color overcoat is a 12% solids suspension of Opadry in water. The color overcoat suspension is sprayed onto the drug overcoated systems until an average wet coated weight of approximately 8 mg per system is achieved.

Next, the color-overcoated systems are clear coated. The clear coat is a 5% solids solution of Opadry in water. The clear coat solution is sprayed onto the color coated cores until an average wet coated weight of approximately 3 mg per system is achieved.

Next, clear-coated systems are coated with approximately 1 g of Carnuaba wax by dispersing the wax over the systems as they tumble in the pan coater.

The dosage form produced by this manufacture is designed to deliver 1 mg of oxycodone hydrochloride USP as an immediate release from an overcoat comprised of 15% oxycodone HCl, USP and 85% hydroxypropyl methylcellulose followed by the controlled delivery of 19mg of oxycodone HCl, USP from the core containing 17.7% oxycodone hydrochloride USP, 78.03% polyethylene oxide possessing a 200,000 molecular weight, 4% polyvinylpyrrolidone possessing a 40,000 molecular weight, 0.02% butylated hydroxytoluene, and 0.25% magnesium stearate. The push composition is comprised 73.7% polyethylene oxide comprising a 7,000,000 molecular weight, 20% sodium chloride, 5% polyvinylpyrrolidone possessing an average molecular weight of 40,000, 1% ferric oxide, 0.05% butylated hydroxytoluene, and 0.25% magnesium stearate. The semi permeable wall is comprised of 99% cellulose acetate of 39.8% acetyl content and 1% polyethylene glycol. The dosage form comprises one passageway, 40 mils (1 mm) on the center of the drug side. The final dosage form contains a color overcoat, a clear overcoat and a wax coat and has a mean release rate of 0.93 mg oxycodone hydrochloride, USP per hour (4.66 %/hr).

### Example 2

### Oxycodone Hydrochloride Biconvex Shaped Bilayer 80 mg System

A dosage form adapted, designed and shaped as an osmotic drug delivery device is manufactured as follows: 32.28 kg of oxycodone hydrochloride, USP , 63.73 kg of polyethylene oxide with average molecular weight of 200,000, are added to a fluid bed granulator bowl. Next, a binder solution is prepared by dissolving 5.45 kg of polyvinylpyrrolidone identified as K29-32 having and average molecular weight of 40,000 in 40 kg of water. The dry materials are fluid bed granulated by spraying with 33.3 kg of binder solution. Next, the wet granulation is dried in the granulator to an acceptable moisture content, and sized using by passing through a 7-mesh screen. The granulation is then transferred to a blender and mixed with 0.02 kg of butylated hydroxytoluene as an antioxidant and lubricated with 0.25 kg of magnesium stearate.

Next, a push composition is prepared as follows: First, a binder solution is prepared by dissolving 15.6 kg of polyvinylpyrrolidone identified as K29-32 having and average molecular weight of 40,000 in 104.4 kg of water. Then, 24 kg of sodium chloride and 1.2 kg of ferric oxide are sized using a Quadro Comil with a 21-mesh screen. The sized materials and 88.44 kg of polyethylene oxide (approximately 2,000,000 molecular weight) are added to a fluid bed granulator bowl. The dry materials are fluidized and mixed while 46.2 kg of binder solution is sprayed from 3 nozzles onto the powder. The granulation is dried in the fluid-bed chamber to an acceptable moisture level. The coated granules are sized using a Fluid Air mill with a 7-mesh screen. The granulation is transferred to a tote tumbler, mixed with 15 g of butylated hydroxytoluene and lubricated with 294 g magnesium stearate.

Next, the oxycodone hydrochloride drug composition and the push composition are compressed into bilayer tablets. First, 250 mg of the oxycodone hydrochloride composition is added to the die cavity and pre-compressed, then, 192 mg of the push composition is added and the layers are pressed into a 13/32" (1.03 cm) diameter round, standard concave, bilayer arrangement.

The bilayered arrangements are coated with a semi-permeable wall. The wall forming composition comprises 99% cellulose acetate having a 39.8% acetyl content and 1 % polyethylene glycol comprising a 3.350 viscosity-average molecular weight. The wall-forming composition is dissolved in an acetone:water (95:5 wt:wt) solvent mixture to make a 5.5% solids solution. The wall-forming composition is sprayed onto and around the bilayered arrangements in a pan coater until approximately 40 mg of membrane is applied to each tablet.

Next, one 40 mil (1 mm) exit passageway is laser drilled through the semi-permeable wall to connect the drug layer with the exterior of the dosage system. The residual solvent is removed by drying for 48 hours as 45 C. and 45% humidity. After drilling, the osmotic systems are dried for 4 hours at 45 C. to remove excess moisture.

Next, the drilled and dried systems are coated with an immediate release drug overcoat. The drug overcoat is a 13% solids aqueous solution containing 1.08 kg of oxycodone HCl, USP and 6.1 kg of hydroxypropyl methylcellulose possessing an average viscosity of 3 centipoise. The drug overcoat solution is sprayed onto the coated systems until an average wet coated weight of approximately 31 mg per system is achieved

Next, the drug-overcoated systems are color overcoated. The color overcoat is a 12% solids suspension of Opadry in water. The color overcoat suspension is sprayed onto the drug overcoated systems until an average wet coated weight of approximately 36 mg per system is achieved.

Next, the color-overcoated systems are clear coated. The clear coat is a 5% solids solution of Opadry in water. The clear coat solution is sprayed onto the color coated systems until an average wet coated weight of approximately 7 mg per system is achieved.

Next, clear-coated systems are coated with approximately 100 ppm of Carnuaba wax by dispersing the wax over the systems as they tumble in the pan coater.

The dosage form produced by this manufacture is designed to deliver 4 mg of oxycodone hydrochloride USP as an immediate release from an overcoat comprised of 15% oxycodone HCl, USP and 85% hydroxypropyl methylcellulose followed by the controlled delivery of 76mg of oxycodone HCl, USP from the core containing 32% oxycodone hydrochloride USP, 63.73% polyethylene oxide possessing a 200,000 molecular weight, 4% polyvinylpyrrolidone possessing a 40,000 molecular weight, 0.02% butylated hydroxytoluene, and 0.25% magnesium stearate. The push composition is comprised 73.7% polyethylene oxide comprising a 7,000,000 molecular weight, 20% sodium chloride, 5% polyvinylpyrrolidone possessing an average molecular weight of 40,000, 1 % ferric oxide, 0.05% butylated hydroxytoluene, and 0.25% magnesium stearate. The semi permeable wall is comprised of 99% cellulose acetate of 39.8% acetyl content and 1% polyethylene glycol. The dosage form comprises one passageway, 40 mils (1 mm) on the center of the drug side. The final dosage form contains a color overcoat, a clear overcoat and a wax coat and has a mean release rate of 4.42 mg oxycodone hydrochloride, USP per hour (5.52 %/hr).

### EXAMPLE 3

### Oxycodone Hydrochloride Capsule Shaped Tablet 23.1 mg System

A dosage form adapted, designed and shaped as an osmotic drug delivery device is manufactured as follow: 23.1 g of oxycodone hydrochloride, 166.5 g of polyethylene oxide) possessing a 200,000 molecular weight, 10.0 g of poly(vinylpyrrolidone) identified as K29-32 having an average molecular weight of 40,000 are added to a Kitchenaid planetary mixing bowl. Next, the dry materials were mixed for 30 seconds.

Then, 80 ml of denatured anhydrous alcohol was slowly added to the blended materials with continuous mixing for approximately 2 minutes. Next, the freshly prepared wet granulation was allowed to dry at room temperature for approximately 18 hours, and passed through a 16-mesh screen. Next, the granulation were transferred to an appropriate container, mixed and lubricated with 1.0 g of stearic acid, then 0.5 g of magnesium stearate.

Next, a push composition is prepared as follows: first, a binder solution is prepared. 5.2 kg of poly(vinylpyrrolidone) identified as K29-32 having an average molecular weight of 40,000 was dissolved in 34.8 kg of water.

22,400 g of sodium chloride was sized using a Quadro Comil with a 21-mesh screen.

Next, 1120 g of ferric oxide was passed through a 40-mesh screen. Then, all the screened materials, 82,540 g of pharmaceutically acceptable polyethylene oxide) comprising a 7,000,000 molecular weight are added to a Glatt Fluid Bed Granulator's bowl. The bowl was attached to the granulator and the granulation process was initiated for effecting granulation. Next, the dry powders were air suspended and mixed. Then, the binder solution was sprayed from 3 nozzles onto the powder. The granulating conditions were monitored during the process as follows: total solution spray rate of 700 g/min; inlet temperature 45 C; and process airflow of 2000 m³/hr.

While spraying the binder solution, the filter bags were shaken for 10 seconds every 30 seconds to unglue any possible powder deposits. At the end of the solution spraying, 43,080 g, the coated granulated particles were continued with the drying process. The machine was turned off, and the coated granules were removed from the granulator.

The coated granules were sized using a Fluid Air mill with a 7 mesh screen. The granulation was transferred to Tote Tumbler, mixed with 56 g of butylated hydroxytoluene and lubricated with 280g stearic acid.

Next, the oxycodone hydrochloride drug composition and the push composition are compressed into bilayer tablets on the Carver Tablet Press. First, 164.3 mg of the oxycodone hydrochloride composition is added to the die cavity and pre-compressed, then, 109.5 mg of the push composition is added and the layers are pressed under a pressure head of approximately ½ a metric ton into a 13/64" (0.516 cm) diameter deep concave longitudinal layered arrangement.

The bilayered arrangements are coated with a subcoat layer. The .wall forming composition comprises 70% hydroxypropyl cellulose having an average molecular weight of 60,000 and 30% poly(vinylpyrrolidone) identified as K29-32 having an average molecular weight of 40,000. The wall-forming composition is dissolved in ethanol to make a 6% solids solution. The wall-forming composition is sprayed onto and around the bilayers in a 12" Vector HiCoater.

The subcoated arrangements are coated with a semi-permeable wall. The wall forming composition comprises 99% cellulose acetate having a 39.8% acetyl content and 1 polyethylene glycol comprising a 3.350 viscosity-average molecular weight. The wallforming composition is dissolved in an acetone:water (95:5 wt:wt) cosolvent to make a 5% solids solution. The wall-forming composition is sprayed onto and around the subcoated arrangements in a 12" Vector HiCoater.

Next, one 35 mil (0.889 mm) exit passageway is mechanically drilled through the semipermeable wall to connect the drug layer with the exterior of the dosage system. The residual solvent is removed by drying for 66 hours as 45 C. and 45% humidity. Next, the osmotic systems are dried for 4 hours at 45 C. to remove excess moisture. The dosage form produced by this manufacture provides 11.5% oxycodone hydrochloride USP, 82.78% poly(ethylene oxide) possessing a 200,000 molecular weight, 4.97% poly(vinylpyrrolidone) possessing a 40,000 molecular weight, 0.5% stearic acid, and 0.25% magnesium stearate. The push composition comprises 73.7% poly(ethylene oxide) comprising a 7,000,000 molecular weight, 20% sodium chloride, 5% poly(vinylpyrrolidone) identified as K29-32 having an average molecular weight of 40,000, 1 % ferric oxide, 0.05% butylated hydroxytoluene, and 0.25% stearic acid. The semipermeable wall comprises 99 wt % cellulose acetate comprising a 39.8% acetyl content and 1 % polyethylene glycol comprising a 3,350 viscosity-average molecular weight. The dosage form comprises one passageway, 35 mils (0.889 mm), and it had an oxycodone hydrochloride mean release rate of 0.77 mg/hr.

### EXAMPLE 4

### Oxycodone Hydrochloride 23.1 g Bilayer System

A dosage form adapted, designed and shaped as an osmotic drug delivery device is manufactured as follows: 23.1 g of oxycodone hydrochloride, 156.5 g of poly(ethylene oxide) possessing a 200,000 molecular weight, 10.0 g of poly(vinylpyrrolidone) identified as K29-32 having an average molecular weight of 40,000 and 10.0 g of sodium chloride are added to a Kitchenaid planetary mixing bowl. Next, the dry materials were mixed for 30 seconds. Then, 80 ml of denatured anhydrous alcohol was slowly added to the blended materials with continuous mixing for approximately 2 minutes. Next, the freshly prepared wet granulation was allowed to dry at room temperature for approximately 18 hours, and passed through a 16-mesh screen. Next, the granulation were transferred to an appropriate container, mixed and lubricated with 1.0 g of stearic acid, then 0.5 g of magnesium stearate.

Next, a push composition is prepared as follows: first, a binder solution is prepared. 3.4 kg of hydroxypropylmethylcellulose possessing an average molecular weight of 11,200 was dissolved in 30.6 kg of water.
27,000 g of sodium chloride was sized using a Quadro Comil with a 21-mesh screen.

Next, 900 g of ferric oxide was passed through a 40-mesh screen. Then, all the screened materials, 57,300 g of pharmaceutically acceptable poly(ethylene oxide) comprising a 2,000,000 molecular weight and 1,800 g of hydroxypropylmethylcellulose possessing an average molecular weight of 11,200 are added to a Glatt Fluid Bed Granulator's bowl. The bowl was attached to the granulator and the granulation process was initiated for effecting granulation. Next, the dry powders were air suspended and mixed. Then, the binder solution was sprayed from 3 nozzles onto the powder. The granulating conditions were monitored during the process as follows: total solution spray rate of 700 g/min; inlet temperature 45^{°} C; and process airflow of 3000 m³/hr.

While spraying the binder solution, the filter bags were shaken for 10 seconds every minute to unglue any possible powder deposits. At the end of the solution spraying, 27,000 g, the coated granulated particles were continued with the drying process. The machine was turned off, and the coated granules were removed from the granulator.

The coated granules were sized using a Fluid Air mill with a 7 mesh screen. The granulation was transferred to Tote Tumbler, mixed with 72 g of butylated hydroxytoluene and lubricated with 225 g magnesium stearate.

Next, the oxycodone hydrochloride drug composition and the push composition are compressed into bilayer tablets on the Carver Tablet Press. First, 113 mg of the oxycodone hydrochloride composition is added to the die cavity and pre-compressed, then, 87 mg of the push composition is added and the layers are pressed under a pressure head of approximately '/2 a metric ton into a 5/16" (0.794 cm) diameter bilayer arrangement.

The bilayered arrangements are coated with a semi-permeable wall. The wall forming composition comprises 99% cellulose acetate having a 39.8% acetyl content and 1 polyethylene glycol comprising a 3.350 viscosity-average molecular weight. The wallforming composition is dissolved in an acetone:water (95:5 wt:wt) cosolvent to make a 5% solids solution. The wall-forming composition is sprayed onto and around the bilayered arrangements in a 12" Vector HiCoater.

Next, one 20 mil (0.508 mm) exit passageway is mechanically drilled through the semipermeable wall to connect the drug layer with the exterior of the dosage system. The residual solvent is removed by drying for 48 hours as 45° C. and 45% humidity. Next, the osmotic systems are dried for 4 hours at 45° C. to remove excess moisture. The dosage form produced by this manufacture provides 18.7% oxycodone hydrochloride USP, 75.55% poly(ethylene oxide) possessing a 200,000 molecular weight, 4.96% poly(vinylpyrrolidone) possessing a 40,000 molecular weight, 0.5% stearic acid, and 0.25% magnesium stearate. The push composition comprises 63.67% poly(ethylene oxide) comprising a 7,000,000 molecular weight, 30% sodium chloride, 5% hydroxypropylmethylcellulose possessing an average molecular weight of 11,200, 1 % ferric oxide, 0.08% butylated hydroxytoluene, and 0.25% magnesium stearate. The semipermeable wall comprises 99 wt % cellulose acetate comprising a 39.8% acetyl content and 1% polyethylene glycol comprising a 3,350 viscosity-average molecular weight. The dosage form comprises one passageway, 20 mils (0.508 mm), and it had an oxycodone hydrochloride mean release rate of 1.1 mg/hr.

### EXAMPLE 5

### Oxycodone Hydrochloride Single Layer Elementary Osmotic Pump System

The system represents the osmotic core containing the drug, surrounded by a semipermeable membrane with a delivery orifice. When exposed to water, the core imbibes water osmotically at a controlled rate, determined by the membrane permeability, and by the osmotic pressure of the core components. Due to a constant internal volume, the system delivers a volume of saturated solution equal to the volume of solvent uptake.

The following shows the prototype system formulation:
Oxycodone HCl 68mg single layer elementary osmotic pump system

### Core:

Oxycodone HCl 18.9%
Mannitol, NF 73.1
Povidone, USP, Ph Eur (K29-32) 1.0%
Crospovidone 3.0%
HPMC, 2910, USP, 5 cps 3.0%
Magnesium Stearate, NF 1.0%
Total Core Weight 378 mg
Semipermeable Membrane
Cellulose Acetate, NF, 320 90%
Polyethylene Glycol 3350, NF, LEO 10%
Solvent: Acetone 88%, Water 12% Coating solution contains 5% solids

A dosage form adapted, designed and shaped as an osmotic drug delivery device is manufactured as follows: 37.8 g of oxycodone hydrochloride, 146.2 g of mannitol, 2.0 g of poly(vinylpyrrolidone) identified as K29-32 having an average molecular weight of 40, 000, and 6.0 g of hydroxypropyl methylcellulose (HPMC) 2910 are added to a Kitchenaid planetary mixing bowl.

Next, the dry materials were mixed for 30 seconds. Then, 70 ml of denatured anhydrous alcohol was slowly added to the blended materials with continuous mixing for approximately 1 minute. Next, the freshly prepared wet granulation was allowed to dry at room temperature for approximately 18 hours, and passes through a 12-mesh screen. Next, the granulation was transferred to an appropriate container, mixed with 6.0 g of crospovidone and blended for 1 minute. Then the granulation was then lubricated with 2.0 g of magnesium stearate for 30 seconds.

The oxycodone HCl drug composition is compressed into single layer tablets on the Carver Tablet Press. First, 378 mg of the oxycodone hydrochloride composition is added to the die cavity and is then compressed under a pressure head of approximately Y 2a metric ton into a 3/8" (0.375 cm) diameter single layer arrangements.

The compressed arrangements are coated with a semi-permeable wall. The wall forming composition comprises 90% cellulose acetate having a 32.0% acetyl content and 10% polyethylene glycol comprising a 3.350 viscosity-average molecular weight. The wall-forming composition is dissolved in an acetone: water (88:12 wt: wt) cosolvent to make a 5% solids solution. The wall-forming composition is sprayed onto and around the bilayered arrangements in a 12" Vector HiCoater.

Next, two 10 mil (0.25 mm) exit passageways (one on each side of the table) are mechanically drilled through the semi-permeable wall to connect the drug layer with the exterior of the dosage system. The residual solvent is removed from the membrane by drying for 48 hours as 45 C. and 45% humidity.

Next, the osmotic systems are dried for 4 hours at 45 C. to remove excess moisture.

### EXAMPLE 6

### Oxycodone Hydrochloride 73.6 mg Bilayer System

A dosage form adapted, designed and shaped as an osmotic drug delivery device is manufactured as follows: 73.6 g of oxycodone hydrochloride, 121.4 g of poly(ethylene oxide) high viscosity with average molecular weight of 200,000, and 4 g of poly(vinylpyrrolidone) identified as K29-32 having an average molecular weight of 40,000 are added to a Kitchenaid planetary mixing bowl. Next, the dry materials were mixed for 30 seconds. Then, 70 ml of denatured anhydrous alcohol was slowly added to the blended materials with continuous mixing for approximately 3 minutes. Next, the freshly prepared wet granulation was allowed to dry at room temperature for approximately 18 hours, and passed through a 12-mesh screen. Next, the granulation was transferred to an appropriate container, mixed and lubricated with 1.0 g of magnesium stearate.

The push composition is prepared as follows: first, a binder solution is prepared. 5.2 kg of poly(vinylpyrrolidone) identified as K29-32 having an average molecular weight of 40,000 was dissolved in 34.8 kg of water.

Next, 22,400 g of sodium chloride was sized using a Quadro Comil with a 21-mesh screen. Next, 1120 g,of ferric oxide was passed through a 21-mesh screen. Then, all the screened materials, 82,540 g of pharmaceutically acceptable poly(ethylene oxide) comprising a 7,000,000 molecular weight are added to a Glatt Fluid Bed Granulator's bowl. The bowl was attached to the granulator and the granulation process was initiated for effecting granulation. Next, the dry powders were air suspended and mixed. Then, the binder solution was sprayed from 3 nozzles onto the powder. The granulating conditions were monitored during the process as follows: total solution spray rate of 700 g/min; inlet temperature 45 C; and process airflow of 2000 m³/hr.

While spraying the binder solution, the filter bags were shaken for 10 seconds every 30 seconds to unglue any possible powder deposits. At the end of the solution spraying, 43,080 g, the coated granulated particles were continued with the drying process. The machine was turned off, and the coated granules where removed from the granulator. The coated granules were sized using a Fluid Air mill with a 7-mesh screen. The granulation was transferred to Tote Tumbler, mixed with 56 g of butylated hydroxytoluene and lubricated with 280 g stearic acid.

The Oxycodone HCl drug composition and the push composition are compressed into bilayer tablets on the Carver Tablet Press. First, 194 mg of the oxycodone hydrochloride composition is added to the die cavity and pre-compressed, then, 149 mg of the push composition is added and the layers are pressed under a pressure head of approximately Y 2a metric ton into a 3/8" (0.375 cm) diameter bilayer arrangement.

The bilayered arrangements are coated with a subcoat layer. The wall forming composition comprises 70% hydroxypropyl cellulose having an average molecular weight of 60,000 and 30% poly(vinylpyrrolidone) identified as K29-32 having an average molecular weight of 40,000. The wall-forming composition is dissolved in ethanol to make a 6% solids solution. The wall-forming composition is sprayed onto and around the bilayers in a 12" Vector HiCoater.

The subcoated arrangements are coated with a semi permeable wall. The wall forming composition comprises 99% cellulose acetate having a 39.8% acetyl content and 1 % polyethylene glycol comprising a viscosity-average molecular weight of 3.350. The wall-forming composition is dissolved in an acetone: water (95:5 wt:wt) cosolvent to make a 5% solids solution. The wall-forming composition is sprayed onto and around the bilayered arrangements in a 12" Vector HiCoater.

Next, one 25 mil (0.64 mm) exit passageway is mechanically drilled through the semi-permeable wall to connect the drug layer with the exterior of the dosage system. The residual solvent is removed by drying for 48 hours as 45 C. and 45% humidity. Next, the osmotic systems are dried for 4 hours at 45 C. to remove excess moisture.

The dosage form produced by this manufacture provides 36.8% oxycodone hydrochloride USP, 60.7% poly(ethylene oxide) possessing a 200,000 molecular weight, 4.0% poly(vinylpyrrolidone) possessing a 40,000 molecular weight, and 0.5% magnesium stearate. The push composition comprises 73.7% poly(ethylene oxide) comprising a 7,000,000 molecular weight, 20% sodium chloride, 5% poly(vinylpyrrolidone) possessing an average molecular weight of 40,000, 1 % ferric oxide, 0.05% butylated hydroxytoluene, and 0.25% magnesium stearate. The semipermeable wall comprises 99 wt% cellulose acetate comprising a 39.8% acetyl content and 1 polyethylene glycol comprising a 3,350 viscosity-average molecular weight.

The dosage form comprises one passageway, 25 mils (0.64 mm), and it had an oxycodone hydrochloride mean release rate of 5 mg/hr.

### EXAMPLE 7

### Oxycodone Hydrochloride Biconvex Shaped 9.5 mg Bilayer System

A dosage form adapted, designed and shaped as an osmotic drug deliverydevice is manufactured as follows: 8.2 g oxycodone hydrochloride, 72.55 g of poly(ethylene oxide) an approximate molecular weight of 200,000 , 4 g of poly(vinylpyrrolidone) identified as K29-32 having an average molecular weight of 40,000, an 15 g of sodium chloride are added to a Kitchen Aid planetary mixing bowl. Next, the dry materials are mixed for 30 seconds. Then, 70 ml of denatured anhydrous alcohol is slowly added to the blended materials with continuous mixing for let approximately 3 minutes. Next, the freshly prepared wet granulation is allowed to dry at room temperature for approximately 18 hours, and passed through a 12 mesh screen. Next, the granulation is transferred to an appropriate container, mixed and lubricated with 0.25 g of magnesium stearate.

The push composition is prepared as follows:
A binder solution is prepared. 5.2 kg of poly(vinylpyrrolidone) identified as K2932 having an average molecular weight of 40,000 is dissolved in 34.8 kg of water.

Next, 22,400 g of sodium chloride is sized using a Quadro Comil with a 21-mesh screen. Next, 1120 g of ferric oxide is passed through a 21-mesh screen. Then, all the screened materials, along with 82,540 g of pharmaceutically acceptable poly(ethylene oxide) comprising a 7,000,000 molecular weight are added to a Glatt Fluid Bed Granulator's bowl. Next, the dry powders are air suspended and mixed in the granulation chamber for approximately 2 minutes. Then, the binder solution is sprayed from 3 nozzles onto the powder. The granulating conditions are monitored during the process as follows: total solution spray rate of 700 g/min; inlet temperature 45 C; and process airflow of 2000 m³/hr.

While spraying the binder solution, the filter bags are shaken for 10 seconds every 30 seconds to dislodge any possible powder deposits. At the end of the solution spraying, 43,080 g, the coated granulated particles are dried in the granulation chamber to a moisture content of approximately 1.5% loss on drying at 75 degrees Celsius. The machine is turned off, and the coated granules are removed from the granulator. The coated granules are sized using a Fluid Air mill with a 7 mesh screen. The granulation is transferred to a Tote Tumbler, mixed with 56 g of butylated hydroxytoluene and lubricated with 280g of stearic acid.

The oxycodone HCl drug composition and the push composition are compressed into bilayer tablets on the Carver Tablet Press. First, 122 mg of the oxycodone hydrochloride composition is added to the die cavity and pre-compressed, then, 94 mg of the push composition is added and the layers are pressed under a pressure head of approximately Y 2a metric ton into a 5/16" (0.312 cm) diameter bilayer arrangement.

The bilayer arrangements are coated with a semi-permeable membrane. The membrane forming composition is 99 % cellulose acetate having a 39.8 % acetyl content and 1% polyethylene glycol comprising a viscosity-average molecular weight of 3.350. The dry materials are dissolved in an acetone: water (95:5 wt:wt) cosolvent to make a 5% solids solution. The membrane-forming composition is sprayed onto and around the bilayered arrangements in a 24" Vector HiCoater.

Next, one 40 mil (1.01 mm) exit passageway is mechanically drilled through the semi-permeable wall to connect the drug layer with the exterior of the dosage system. The residual solvent is removed by drying for 48 hours as 45 C and 45% humidity. Next, the osmotic systems are dried for 4 hours at 45 C to remove excess moisture.

The dosage form produced by this manufacture provides 8.2% oxycodone hydrochloride USP, 72.55 % poly(ethylene oxide) possessing a 200, 000 molecular weight, 4.0°I poly(vinylpyrrolidone) possessing a 40,000 molecular weight, and 0.25% magnesium stearate. The push composition comprises 73.7% poly(ethylene oxide) comprising a 7,000,000 molecular weight, 20% sodium chloride, 5% poly(vinylpyrrolidone) possessing an average molecular weight of 40,000, 1% ferric oxide, 0.05% butylated hydroxytoluene, and 0.25% magnesium stearate. The semipermeable wall comprises 99 wt % cellulose acetate comprising a 39.8°/ acetyl content and 1 % polyethylene glycol comprising a 3,350 viscosity-average molecular weight. The dosage form comprises one passageway, 40 mils (1.01 mm), and with 35 mg of membrane, it delivers 9.5 mg of oxycodone hydrochloride at a mean release rate of 0.5 mg/hr in a 71.6% zero order profile.

Also described herein is a method for administrating 1 to 500 mg of oxycodone to a patient in need of pain relief. The method, in one administration, comprises admitting orally into the patient 1 to 500 mg of a oxycodone selected from the group consisting of oxycodone base or oxycodone salt that is administered from a therapeutic composition, 20 to 375 mg of poly(alkylene oxide) having a 50,000 to 750,000 molecular weight, 0.01 to 25 mg of poly(vinyl pyrrolidone) having a 5,000 to 350,000 molecular weight, and 0.01 to 10 mg of a lubricant, which composition provides oxycodone therapy over an extended period of time.

Also described is a method for administrating 1 to 500 mg of oxycodone to a patient admitting orally 1 to 500 mg of oxycodone to the patient, which is administered from a dosage form comprising a semipermeable wall permeable to aqueous-biological fluid and impervious to the passage of oxycodone. The semipermeable wall surrounds an internal space or compartment comprising a oxycodone drug composition and a push composition. The oxycodone drug composition comprises 1 to 500 mg of oxycodone, 20 to 375 mg of poly(alkylene oxide) having a 50,000 to 750,000 molecular weight, 0.01 to 25 mg of poly(vinylpyrrolidone) having a 5,000 to 350,000 molecular weight, and 0 to 10 mg of a lubricant. The push composition comprises 20 to 375 mg of a hydrogel polymer, such as a poly(alkylen oxide) of 1,000,000 to 10,000,000 molecular weight, 0 to 75 mg of an osmagent, 0 to 75 mg of hydroxyalkylcellulose, 0.01 to 5.5 mg of a colorant, 0.01 to 10 mg of a lubricant, and 0 to 10 mg of an antioxidant; and exit means in the semipermeable wall for delivering the oxycodone from the dosage form by imbibing fluid through the semipermeable wall into the dosage form, causing the oxycodone composition to become dispensable and causing the push composition to expand and push the oxycodone composition through the exit, whereby, through the combined operations of the dosage form, the oxycodone is delivered at a therapeutically effective dose at a controlled rate over a sustained period of time.

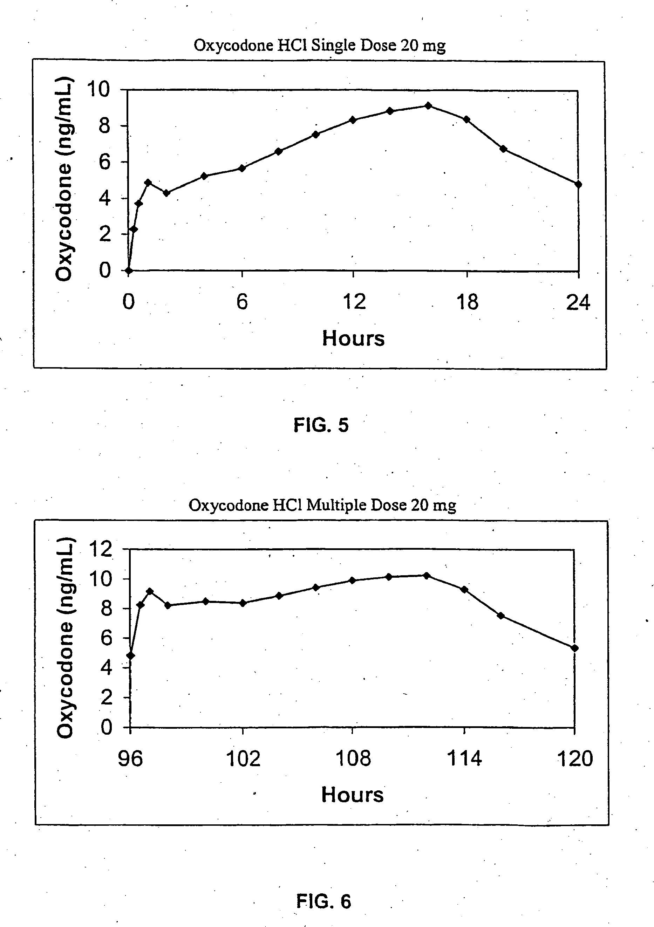

Figure 5 depicts the mean plasma oxycodone concentration profiles for oxycodone treatment on day one. The osmotically controlled extended-release dosage form results are illustrated by the solid line with black circles. This dosage form was administered once-a-day, and it comprised 20 mg of oxycodone.

Figure 6 depicts the mean plasma oxycodone concentration following oxycodone treatment on days four and five, steady state. In Figure 6, the solid line with black circles denotes the plasma profile for the osmotic dosage form administered once-a-day, which comprised 20 mg of oxycodone.
There is disclosed methods for administrating oxycodone to a patient, and methods for producing a plasma concentration of oxycodone. The method provides for admitting orally to a patient a dosage form that administers at a controlled rate, over a continuous time up to 24 hours, oxycodone for its intended therapy. The method also comprises administering orally to a patient a therapeutic dose of oxycodone from a single dosage form that administers the oxycodone over 24 hours. The method further comprises administering oxycodone for producing a first oxycodone concentration in the plasma, a second, elevated oxycodone concentration in the plasma, and a third, continuous oxycodone concentration in the plasma.

## Claims

1. A sustained release oral dosage form for once-a-day controlled delivery of oxycodone comprising:
(a) a core which comprises:
(i) an osmotic agent; and
(ii) oxycodone and/or one or more pharmaceutically-acceptable acid addition salts thereof (the compound);
(b) a semipermeable membrane enveloping the core;
(c) an exit orifice through the semipermeable membrane which communicates with the core so as to alow release of the compound to the environment; and
(d) an immediate release coating which comprises 0.5 to 75mg of the compound and 0.5 to 275mg of a pharmaceutically acceptable carrier selected from the group consisting of alkylcellulose, hydroxyalkylcellulose and hydroxypropylalkylcellulose.

2. A sustained release oral dosage form according to any claim 1 wherein the core comprises a polyalkylene oxide.

3. A sustained release oral dosage form according to claim 1 or 2 wherein the core comprises:
(i) a first drug layer which comprises the compound, and
(ii) a second expandable layer which comprises the osmotic agent and does not comprise the compound.

## Patentansprüche

1. Orale Dosierungsform zur dauerhaften Freisetzung einer einmal täglichen kontrollierten Abgabe von Oxykodon umfassend:
(a) einen Kern, die
(i) ein osmotisches Mittel; und
(ii) Oxykodon und/oder ein oder mehrere pharmazeutisch akzeptable Säure-Additions-Salze davon (die Verbindung) umfaßt;
(b) eine semipermeable Membran, die den Kern umhüllt;
(c) eine Ausgangsöffnung durch die semipermeable Membran, die mit dem Kern kommuniziert, um die Verbindung langsam in die Umgebung feizusetzen; und
(d) eine sofort freisetzende Beschichtung, die 0,5 bis 75 mg der Verbindung und 0,5 bis 275 mg eines pharmazeutisch akzeptablen Trägers umfaßt, der ausgewählt wird aus der Gruppe bestehend aus Alkylcellulose, Hydroxyalkylcellulose und Hydroxypropylcellulose.

2. Orale Dosierungsform zur dauerhaften Freisetzung nach Anspruch 1, bei der der Kern ein Polyalkylenoxid umfaßt.

3. Orale Dosierungsform zur dauerhaften Freisetzung nach einem der Ansprüche 1 oder 2, bei der der Kern umfaßt
(i) eine erste Arzneimittelschicht, die die Verbindung umfaßt, und
(ii) eine zweite expandierbare Schicht, die das osmotische Mittel und nicht die Verbindung umfaßt.

## Revendications

1. Forme posologique orale à libération entretenue pour une administration contrôlée une fois par jour d'oxycodone, comprenant :
(a) un noyau qui comprend :
(i) un agent osmotique ; et
(ii) de l'oxycodone et/ou un ou plusieurs sels d'addition avec les acides, pharmaceutiquement acceptables, de celle-ci (le composé) ;
(b) une membrane semi-perméable enveloppant le noyau ;
(c) un orifice de sortie à travers la membrane semi-perméable qui communique avec le noyau de façon à permettre une libération du composé dans l'environnement ; et
(d) un revêtement à libération immédiate qui comprend de 0,5 à 75 mg du composé et de 0,5 à 275 mg d'un support pharmaceutiquement acceptable choisi dans le groupe constitué par l'alkylcellulose, l'hydroxyalkylcellulose et l'hydroxypropylalkylcellulose.

2. Forme posologique orale à libération entretenue selon la revendication 1, dans laquelle le noyau comprend un poly(oxyde d'alkylène).

3. Forme posologique orale à libération entretenue selon l'une des revendications 1 ou 2, dans laquelle le noyau comprend :
(i) une première couche de médicament qui comprend le composé ; et
(ii) une seconde couche expansible qui comprend l'agent osmotique et ne comprend pas le composé.
